# EUROPEAN PATENT APPLICATION

(11) **EP 3 827 824 A1**
(43) Date of publication of application: **02.06.2021**
(21) Application number: 19211521.0
(22) Date of filing: 26.11.2019
(51) Int. Cl.: A61K 31/353, A61K 31/122, A61K 31/415, A61K 31/513, A61K 31/52, A61P 31/14, A61P 43/00

(54) **TREATMENT OF CONGENITAL ZIKA VIRUS SYNDROME**

(71) Applicant: Hercules Pharmaceuticals B.V., 2333 BD Leiden (NL); The Brigham and Women's Hospital, Inc., Boston, MA 02115 (US)
(72) Inventor: QUINTANA, Francisco Javier, BOSTON, 02115 (US)
(74) Representative: Kehoe, Laura Ellen

(57) **Abstract**

The invention is in the field of medical interventions, more in particular the treatment, amelioration or prevention of viral infections, such as flavivirus infections, more in particular of infections with ZIKA virus. More in particular, the present description provides means and methods for the treatment, amelioration and prevention of congenital ZIKA virus syndrome. Even more in particular, the invention relates to an antagonist of the aryl hydrocarbon receptor (AHR) or a pharmaceutically acceptable salt thereof for use in the treatment, amelioration or prevention of congenital ZIKA Virus (ZIKV) syndrome.

## Description

### Field of the invention

The invention is in the field of medical interventions, more in particular the treatment, amelioration or prevention of viral infections, such as flavivirus infections, more in particular of infections with ZIKA virus. More in particular, the present description provides means and methods for the treatment, amelioration and prevention of congenital ZIKA virus syndrome.

### Background of the invention

Zika virus (ZIKV) is a flavivirus linked to multiple birth defects including fetal microcephaly, known as congenital ZIKV syndrome. The identification of host factors involved in ZIKV replication may guide efficacious therapeutic interventions. In genome-wide transcriptional studies we found that ZIKV activates the aryl hydrocarbon receptor (AHR). AHR activation by ZIKV limits the production of type I interferons involved in innate anti-viral immunity. Moreover, ZIKV-triggered AHR activation suppresses intrinsic immunity driven by the promyelocytic leukemia protein (PML), which we found limits ZIKV replication. AHR inhibition suppressed the replication of multiple ZIKV strains, and also of the related flavivirus, dengue virus. Finally, AHR inhibition with nanoparticle-delivered AHR antagonists and specific-inhibitors developed for human use limited ZIKV replication in vivo and ameliorated newborn microcephaly in a murine model. In summary, we identified AHR as a host factor for ZIKV replication, and PML as a driver of anti-ZIKV intrinsic immunity.

Zika virus (ZIKV) is an enveloped, positive-sense single stranded RNA virus in the Flavivirus genus of the *Flaviviridae* family; this genus includes other viruses of public health concern such as dengue (DENV), yellow fever and Japanese encephalitis^{1,2}. Human ZIKV infection was first reported in the 1950s in Africa³, but less than 20 cases had been reported until 2007 when the first ZIKV outbreak in Yap, Gabon and the South Pacific occurred⁴. In 2014 the virus spread to the Americas and since then, 87 countries reported the emergence of ZIKV⁵⁻⁷. The symptoms of ZIKV infection are generally mild and most ZIKV infected individuals do not develop disease. However, following its re-emergence in 2013, ZIKV has been linked to a congenital Zika syndrome characterized by fetal brain abnormalities⁸⁻¹⁰, and also to Guillain-Barre syndrome¹¹. The development of effective therapies against ZIKV requires a better understanding of virus-host interactions and anti-viral immunity. In addition to innate and adaptive immunity, cell intrinsic defense mechanisms collectively known as intrinsic immunity participate in the control of viral infections¹³. Intrinsic immunity is mediated by proteins that display antiviral activity and block viral replication at multiple levels, such as the products of *2'-5'-oligoadenylate synthetase 1b (Oaslb)*¹⁴*, interferon stimulated exonuclease gene 20*¹⁵*(ISG20)* and *promyelocytic leukemia*¹⁶ *(Pml)* genes. Little is known, however, about restriction factors that limit ZIKV replication. Despite its association to these severe outcomes, no therapy is currently approved for the treatment of ZIKV or other flavivirus infection¹², highlighting the unmet clinical need for approaches to manage emerging flavivirus outbreaks.

### Summary of the invention

This problem has been solved by the present invention in that compounds and compositions are provided for use in the treatment of congenital ZIKA virus syndrome. Also provided is a method for the treatment or amelioration or prevention of ZIKA virus infection. In other terms, the invention provides an antagonist of the aryl hydrocarbon receptor (AHR) or composition comprising such an antagonist or a pharmaceutically acceptable salt thereof for use in the treatment, amelioration or prevention of congenital ZIKA Virus (ZIKV) syndrome.

### Legend to the figures

Figure 1: AHR signaling is activated by ZIKV infection. mRNA expression of genes AHR (A), CYP1A1 (B) and CYP1B1 (C) determined by qPCR at different time points of ZIKV infection of HepG2 cells (moi = 1). Expression values are relative to mock-infected cells. Data are mean ± s.e.m. from at least three independent experiments. P values were determined using a one-sample t-test.
Figure 2: *TDO2* expression determined by qPCR in ZIKV-infected HepG2 cells (moi = 1). Expression values are relative to mock-infected cells. Data represent the mean + s.e.m. from three independent experiments. P values were determined using a one-sample t-test.
Figure 3: ELISA quantification of Kyn (A) and Trp (B) in culture supernatants 48 h p.i. of HepG2 cells with ZIKV (moi = 1). Data represent the mean + s.e.m. from three independent experiments. P values were determined by Student's t-test.
Figure 4: AHR (A), CP1A1 (B), IDO1 (C) and TDO2 (D) mRNA expression determined by qPCR in ZIKV-infected NPCs (moi = 1). Expression values are relative to mock-infected cells. Data are mean ± s.e.m. from three independent experiments. P values were determined using a one-sample t-test.
Figure 5: Effect of moi (0.1 or 10) on mRNA expression of AHR (A), CP1A1 (B), IDO1 (C) and TDO2 (D) in ZIKV-infected NPCs 48 h p.i. Expression values are relative to mock-infected cells. Data are mean ± s.e.m. from at least three independent experiments. P values were determined by Student's t-test.
Figure 6: Kyn in supernatants of ZIKV-infected NPCs 48 h p.i. Data represent the mean + s.e.m. from three independent experiments. P values were determined by a one-way ANOVA followed by Tukey's post-hoc test.
Figure 7A-K: AHR signaling boosts ZIKV replication in vitro. Flow cytometry quantification of HepG2 cells positive for flavivirus antigen. HepG2 cells were pre-treated with different concentrations of I3S, CH223191 or DMSO (vehicle) and infected with ZIKV (moi = 1) for 48 h. Plots shown are representative of one experiment. Bar graphs (7C) represent the mean + s.e.m. of flavivirus antigen positive cells from at least three independent experiments. P values were determined by a one-way ANOVA followed by Tukey's post-hoc test.
Figure 8: Quantification of infectious virus particles in the supernatants of HepG2 cells pre-treated with CH2213191 or vehicle-treated using a standard plaque assay in Vero cells . Data represent the mean + s.e.m. from three independent experiments. P values were determined by a one-way ANOVA followed by Tukey's post-hoc test.
Figure 9: Toxicity of I3S and CH223191. Cell viability after treatment for 24 h with different concentrations of I3S (A) and CH223191 (B) evaluated by MTT assay. Preferred dilutions are highlighted.
Figure 10: AHR modulation in NPCs. NPCs were treated with I3S or a combination of I3S and CH223191. 24 h after treatment, NPCs were harvested for RNA extraction followed by qPCR analysis of AHR (A), CYP1A1 (B), IDO1 (C) and TDO2 (D). Data represent the mean + s.e.m. from three independent experiments. P values were determined by Student's t-test.
Figure 11: Quantification of infectious virus particles in AHR or control knock down HepG2 cells. HepG2 cells were transfected with scrambled-siRNA or AHR-siRNA for 48 h, infected with ZIKV (moi = 0.1) and 48 h p.i. supernatants were harvested for a standard plaque assay in Vero cells. Data represent the mean + s.e.m. from four independent experiments. P values were determined by Student's t-test.
Figure 12: Quantification of infectious virus particles in AHR or control knock down HepG2 cells. HepG2 cells were transfected with siRNAs as indicated for 48 h, infected with ZIKV (moi = 0.1) and 48 h p.i. supernatants were harvested for a standard plaque assay in Vero cells. Data represent the mean + s.e.m. from four independent experiments. P values were determined by Student's t-test
Figure 13: Quantification of infectious virus particles in the supernatants of human NPC cells pre-treated with CH2213191 or vehicle-treated using a standard plaque assay in Vero cells . Data represent the mean + s.e.m. from three independent experiments. P values were determined by a one-way ANOVA followed by Tukey's post-hoc test.
Figure 14: Quantification of infectious virus particles in the supernatants of HepG2 cells 48 h p.i. after infection with ZIKV INEVH116141 (Argentina) using a standard plaque assay in Vero cells. Data represent the mean + s.e.m. from three independent experiments. P values were determined by a one-way ANOVA followed by Tukey's post-hoc test.
Figure 15: Quantification of infectious virus particles in the supernatants of HepG2 cells 48 h p.i. after infection with ZIKV BeH815744 (Brazil) using a standard plaque assay in Vero cells. Data represent the mean + s.e.m. from three independent experiments. P values were determined by a one-way ANOVA followed by Tukey's post-hoc test.
Figure 16: Quantification of infectious virus particles in the supernatants of HepG2 cells 48 h p.i. after infection with ZIKV MR 766 (Uganda) using a standard plaque assay in Vero cells. Data represent the mean + s.e.m. from three independent experiments. P values were determined by a one-way ANOVA followed by Tukey's post-hoc test..
Figure 17:Q-PCR quantification of ZIKV RNA in HepG2 cells treated with CH223191 or DMSO (vehicle) before infection, during infection, or after the infection with ZIKV (moi = 3). 2 h p.i., cells were harvested for RNA extraction and qPCR analysis to determine the level of ZIKV RNA. Data represent the mean + s.e.m. from three independent experiments. P values were determined by Student's t-test.
Figure 18: AHR limits IFN-I dependent and PML-driven intrinsic immunity to ZIKV. NF-κB activation in ZIKV-infected and CH223191-treated cells determined by Western Blot. HepG2 cells were pre-treated with CH223191 and infected with ZIKV. 48 h later cells were harvested for WB analysis. Data represent the mean + s.e.m. from three independent experiments. P values were determined by a one-way ANOVA followed by Tukey's post-hoc test.
Figure 19: mRNA expression determined by qPCR in ZIKV-infected HepG2 pre-treated with CH223191. Expression of cellular genes shown relative to non-treated, mock-infected cells. RNA level of ZIKV is relative to ZIKV-infected cells immediately harvested after infection. Data represent the mean + s.e.m. from three independent experiments. P values were determined by Student's t-test
Figure 20: mRNA expression determined by qPCR in ZIKV-infected human NPCs pre-treated with CH223191. Expression of cellular genes shown relative to non-treated, mock-infected cells. RNA level of ZIKV is relative to ZIKV-infected cells immediately harvested after infection. Data represent the mean + s.e.m. from three independent experiments. P values were determined by Student's t-test
Figure 21: Flow cytometry quantification of Vero cells expressing flavivirus antigen. Vero cells were pre-treated with CH223191 or DMSO (vehicle) and infected with ZIKV (moi = 1). Flow cytometry plots are representative of one experiment. Bar graphs shown on the right represent the mean + s.e.m. of virus antigen positive cells from three independent experiments. P values were determined by a one-way ANOVA followed by Tukey's post-hoc test.
Figure 22 Effect of inhibitors of IFN signaling on ZIKV replication determined 24 h p.i. by plaque assay. HepG2 cells were pre-treated with DMSO (vehicle), JAK I Inhibitor, TSA, or NaF for 24 h and infected with ZIKV. Data represent the mean + s.e.m. from three independent experiments. P values were determined by a one-way ANOVA followed by Tukey's post-hoc test.
Figure 23: Quantification of infectious virus particles in supernatants of Vero cells pre-treated with CH223191 or DMSO (vehicle) and infected with ZIKV (moi=1). 48 h p.i. supernatants were harvested for a standard plaque assay in Vero cells. Data represent the mean + s.e.m. from three independent experiments. P values were determined by a one-way ANOVA followed by Tukey's post-hoc test.
Figure 24: Effect of CH223191 on HepG2 cells treated with JAK I inhibitor. HepG2 cells were treated with JAK I Inhibitor and CH223191 as indicated in the figure, and then infected with ZIKV (moi = 0.1), and 24 h p.i. supernatants were harvested for a standard plaque assay in Vero cells
Figure 25: Schematic representation of the *Pml* promoter. Predicted binding sites to AHR and NF-κB were determined using Mulan software.
Figure 26: ChIP analysis of AHR and NF-KB binding sites in the *Pml* promoter. HepG2 cells were treated with CH223191 and infected with ZIKV as indicated in the figure. 48 h p.i. cells were fixed and ChIP was performed using AHR and NF-KB-specific antibodies or non-specific IgG. Data are mean + s.e.m. and are representative of two independent experiments.
Figure 27: ChIP analysis of AHR binding to site 2 in *Pml* promoter*.* HepG2 cells were treated with CH223191 and infected with ZIKV as indicated in the figure. 48 h p.i. cells were fixed and ChIP was performed using AHR and NF-KB-specific antibodies or non-specific IgG. Data are mean + s.e.m. and are representative of two independent experiments
Figure 28: Luciferase assay using a construct coding for the *PML* promoter cloned upstream of the reporter gene *Gaussia* Luciferase. HEK-293 cells were pre-treated with CH223191 and transfected with plasmids as indicated. 24 h post transfection, cells were harvested for quantification of Gaussia Luciferase and TK-Renilla activity. Data represent the mean + s.e.m. from three independent experiments. P values were determined by a one-way ANOVA followed by Tukey's post-hoc test.
Figure 29: HeLa cells were transfected with a plasmid encoding for GFP (control) or PML-shRNAs and pre-treated with CH223191 as indicated in the figure. 48 h post transfection, cells were infected with ZIKV (moi = 1) and 24 h p.i. supernatants were harvested for quantification of the number of infectious virus particles by a standard plaque assay. Viral titers were normalized to non-transfected cells. Data represent the mean + s.e.m. from three independent experiments. P values were determined by a one-way ANOVA followed by Tukey's post-hoc test.
Figure 30: Effect of PMLIII or PMLIV on ZIKV production. HepG2 cells were transfected with plasmids encoding for nlsGFP (control), PMLIII-RFP or PMLIV-RFP. 48 h p.t. cells were infected with ZIKV (moi = 0.1) and 24 h p.i., supernatants were harvested for quantification of the number of infectious virus particles by a standard plaque assay. Data represent the mean + s.e.m. from three independent experiments. P values were determined by a one-way ANOVA followed by Tukey's post-hoc test.
Figure 31: Nanoliposome-delivered AHR antagonist limits ZIKV replication and microcephaly *in vivo.* HEK293 cells were transfected with an AHR-responsive reporter (pGud-Luc) and treated as indicated with Kyn, NLP, NLP_{ANT} or CH-223191 (*n*= 3, representative of three independent experiments, one-way ANOVA followed by Tukey's multiple comparisons test; normalized to Vehicle).
Figure 32: Bone marrow-derived DCs were treated as indicated with Kyn, NLP, NLP_{ANT} or CH-223191 and the expression of the AHR-target gene *Cyp1b1* was determined by qPCR (n = 3, representative of three independent experiments, one-way ANOVA followed by Tukey's multiple comparisons test; normalized to Vehicle).
Figure 33: Pictures of newborn pups from ZIKV infected mothers treated with NLP (Top) or NLP_{ANT} (Bottom).
Figure 34: Pictures of brains from pups shown in figure 33.
Figure 35: ZIKV viral load in brain of pups from infected pregnant SJL mice treated with NLP, NLPANT or from non-infected controls. Data represent the mean + s.e.m. from three independent experiments. p values were determined by Student's t-test. *p<0.05. p values were determined by One-way ANOVA. *p<0.05. "p<0.01. ***p<0.001.
Figure 36: ZIKV viral load in spleen of pups from infected pregnant SJL mice treated with NLP, NLP_{ANT} or from non-infected controls. Data represent the mean + s.e.m. from three independent experiments. p values were determined by Student's t-test. *p<0.05. p values were determined by One-way ANOVA. *p<0.05. "p<0.01. ***p<0.001.
Figure 37: Macroscopic analysis of pups at birth; weight. Data represent the mean + s.e.m. from three independent experiments.
Figure 38: Macroscopic analysis of pups at birth; Crown-rump. Data represent the mean + s.e.m. from three independent experiments.
Figure 39: Macroscopic analysis of pups at birth; biparietal. Data represent the mean + s.e.m. from three independent experiments.
Figure 40: Macroscopic analysis of pups at birth; Skull length. Data represent the mean + s.e.m. from three independent experiments.
Figure 41: Macroscopic analysis of pups at birth; Cranial height. Data represent the mean + s.e.m. from three independent experiments.
Figure 42: Brain histology of hippocampal region from ZIKV and ZIKV+NLP_{ANT} pups. Brackets indicate cortical thickness.
Figure 43: Transcriptional effects of AHR antagonism during ZIKV infection *in vivo.* mRNA expression determined by RNA-Seq analysis of CNS fetal samples from NLP- or NLP_{ANT}-treated ZIKV infected mice.
Figure 44: Cell death gene expression transcriptional signature. Pooled brain RNA from 3 pups from NLP and NLP_{ANT} treated mothers were used for PCR array analysis for Cell Death Pathway Finder RT² Profiler (Qiagen). AHR blockade with NLP_{ANT} reduced the expression of several pro-apoptotic and autophagy-related genes. Data are expressed as fold change comparing two groups.
Figure 45: AHR antagonist HP163. SUM149 cells which express constitutive AHR activity, were transfected with the AHR-driven pGudLuc reporter construct and treated with vehicle (0.1% DMSO) or 20 µM HP163. Twenty-four hours later cells were assayed for pGudLuc (luciferase) activity and normalized to the CMV-green fluorescence signal. Data were obtained from two independent experiments, each with four replicates. **p<0.01.
Figure 46: Murine MOC1 oral cancer cells were treated with vehicle or 20 µM HP163 for 30 min and then treated with 0.1% DMSO, 100 µM Kyn or 1 nM TCDD for 1 hour. Nuclear and cytoplasmic extracts were prepared and AHR protein quantified by Western immunoblotting. Data presented are representative of three independent experiments.
Figure 47: Quantification of Western immunoblotting bands from the three experiments described in figure 46. Data are presented as average + SE. **p<0.01 as compared with DMSO controls within the respective vehicle, Kyn and TCDD groups. # p<0.05 as compared with cells treated only with DMSO. + p=0.05 as compared with the DMSO controls.
Figure 48: A novel oral AHR antagonist limits ZIKV replication and microcephaly *in vivo.* Histological analysis of hippocampal region from control- and HP163-treated fetuses. Asterisks indicate ventricle dilatation and brackets indicate cortical thickness.
Figure 49 Brains from pups used in figure 48.
Figure 50: ZIKV viral load in spleen from pups of infected pregnant SJL mice treated with HP163 or vehicle. Data represent the mean + s.e.m. from three independent experiments. p values were determined by Student's t-test. *p<0.05. "p<0.01. ***p<0.001.
Figure 51: ZIKV viral load in brain from pups of infected pregnant SJL mice treated with HP163 or vehicle. Data represent the mean + s.e.m. from three independent experiments. p values were determined by Student's t-test. *p<0.05. "p<0.01. ***p<0.001.
Figure 52: ZIKV viral load in eyes from pups from infected pregnant SJL mice treated with HP163 or vehicle. Data represent the mean + s.e.m. from three independent experiments. p values were determined by Student's t-test. *p<0.05. "p<0.01. ***p<0.001.
Figure 53: ZIKV viral load in placenta from infected pregnant SJL mice treated with HP163 or vehicle. Data represent the mean + s.e.m. from three independent experiments. p values were determined by Student's t-test. *p<0.05. "p<0.01. ***p<0.001.
Figure 54: Macroscopic evaluation of pups at birth for weight measurements. Data represent the mean + s.e.m. from three independent experiments. p values were determined by Student's t-test. *p<0.05. "p<0.01. ***p<0.001.
Figure 55: Macroscopic evaluation of pups at birth for crown-rump measurements. Data represent the mean + s.e.m. from three independent experiments. p values were determined by Student's t-test. *p<0.05. "p<0.01. ***p<0.001.
Figure 56: Macroscopic evaluation of pups at birth for skull length measurements. Data represent the mean + s.e.m. from three independent experiments. p values were determined by Student's t-test. *p<0.05. "p<0.01. ***p<0.001.
Figure 57: Macroscopic evaluation of pups at birth for biparietal measurements. Data represent the mean + s.e.m. from three independent experiments. p values were determined by Student's t-test. *p<0.05. "p<0.01. ***p<0.001.
Figure 58: Macroscopic evaluation of pups at birth for cranial height measurements. Data represent the mean + s.e.m. from three independent experiments. p values were determined by Student's t-test. *p<0.05. "p<0.01. ***p<0.001.
Figure 59: Immunofluorescence analysis for Nestin (pe), ZIKV (fitc) and nuclei (dapi) from brains of fetuses from SJL mothers infected with ZIKV and treated or not treated with HP163. Images are merged on the right panel. Quantification panels are shown on the right.
Figure 60: Immunofluorescence analysis for Iba-1(pe) and nuclei (dapi) in brains of the same groups as in figure 59. Quantification panel is showed on the right.
Figure 61: AHR modulation by small molecules in A549 cells. A549 cells were treated with the indicated concentrations of I3S (A) or CH223191 (B) for 24 h. Then, cells were harvested for RNA extraction and qPCR analysis of AHR and CYP1A1.
Figure 62: AHR activation boosts DENV replication. A549 cells were pretreated with the indicated concentrations of I3S for 24 h and infected with DENV-2 (moi = 0.1). 48 h p.i. supernatants were harvested for plaque assay.
Figure 63: AHR modulation affects DENV replication Infectious virus particles in the supernatants of A549 cells pre-treated with CH2213191 and infected with DENV-1 (A), DENV-2 (B), DENV-3 (C) or DENV-4 (D) (moi = 0.1). At 24 h p.i. supernatants were harvested for plaque assay.
Figure 64: A549 cells positive for flavivirus antigen determined by immunofluorescence. Cells infected with DENV-2 from figure 63 were fixed, stained against flavivirus envelope protein and visualized under a fluorescence microscope. Images of random fields of view were acquired and the number of cells positive for flavivirus antigen was counted using FIJI.
Figure 65: RNA expression determined by qPCR. A549 cells were infected with DENV-2 (moi = 0.1) and 48 h p.i. they were harvested for qPCR analysis.
Figure 66: Infectious virus particles in supernatants of A549 cells transfected with scrambled-siRNA or AHR-siRNA.
Figure 67: Infectious virus particles in supernatants of A549 cells transfected with scrambled-siRNA, AHR-siRNA or other relevant siRNAs indicated in the figure.. Cells were transfected for 48 h, infected with DENV-2 (moi = 0.1) and 48 h p.i. supernatants were harvested for a standard plaque assay in Vero cells. Data represent the mean + s.e.m. from four independent experiments.

### Detailed description of the invention

Here we report that aryl hydrocarbon receptor (AHR) activation by ZIKV infection limits the production of type I interferons (IFN-I), NF-κB signaling and intrinsic immunity mechanisms that suppress viral replication. Conversely, *in vivo* treatment with AHR antagonists suppresses viral replication in fetal tissue, reducing overall fetal malformations linked to congenital Zika syndrome. Collectively, these studies define AHR as a host enabling factor for ZIKV replication and PML as a driver of anti-ZIKV intrinsic immunity, and identify AHR as a candidate therapeutic target for the management of ZIKV infection.

### AHR signaling is activated by ZIKV infection

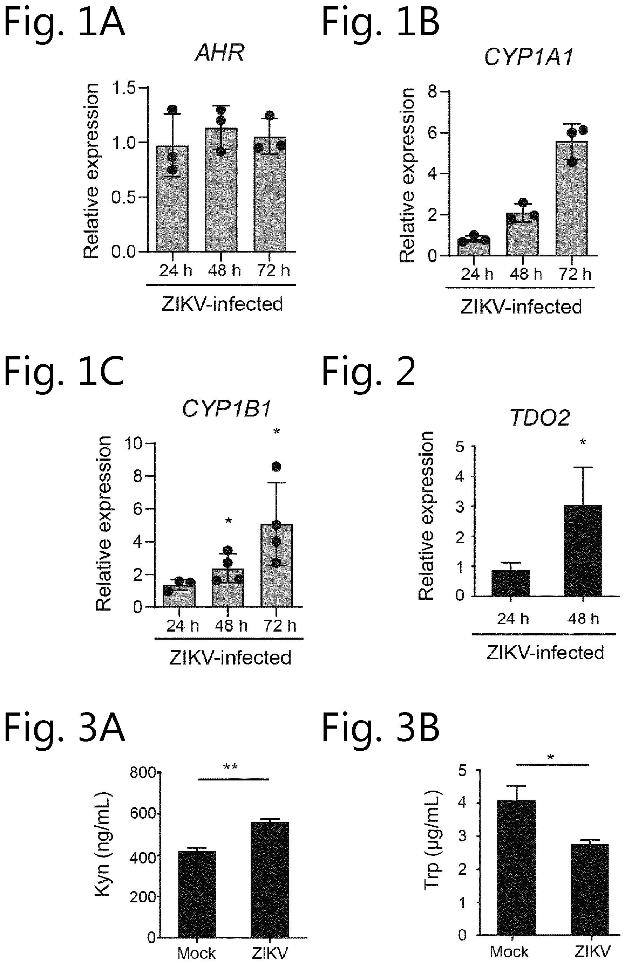

The liver has been identified as a primary target of human ZIKV infection *¹⁷*. To identify molecular mechanisms associated with ZIKV replication, we analyzed by RNA-seq the expression profile of liver-derived human HepG2 cells infected with ZIKV. We detected 179 differentially expressed genes linked to cellular processes such as protein translation, cell cycle control, energy metabolism and autophagy (Table 1). These processes have been shown to be targeted by ZIKV and other flaviviruses to promote their replication¹⁸⁻²⁰. In addition, we identified the transcription factor aryl hydrocarbon receptor (AHR) as a candidate upstream regulator of the transcriptional response of HepG2 cells to ZIKV infection by pathway overrepresentation analysis. Indeed, in validation studies on independent samples we detected increased expression of the AHR transcriptional targets *CYP1A1* and *CYP181* in ZIKV infected HepG2 cells (Fig. 1); the expression of AHR itself was unaffected. These findings prompted us to investigate the role of AHR in ZIKV replication.

AHR is a ligand-activated transcription factor whose transcriptional activity requires both AHR expression and the presence of AHR agonists²¹. Kynurenine (Kyn) is a tryptophan (Trp)-derived AHR ligand produced in the context of cancer and inflammation by the enzymes indoleamine 2,3-dioxigenase 1 and 2 (IDO1 and IDO2) and tryptophan 2,3-dioxigenase (TDO)^{22, 23}. We did not detect *IDO1* nor *IDO2* expression in ZIKV-infected HepG2 cells. However, ZIKV infection upregulated *TDO* expression and increased Kyn levels in culture supernatants, while it decreased the levels of Trp used as a substrate by TDO (Figs. 2 and 3).

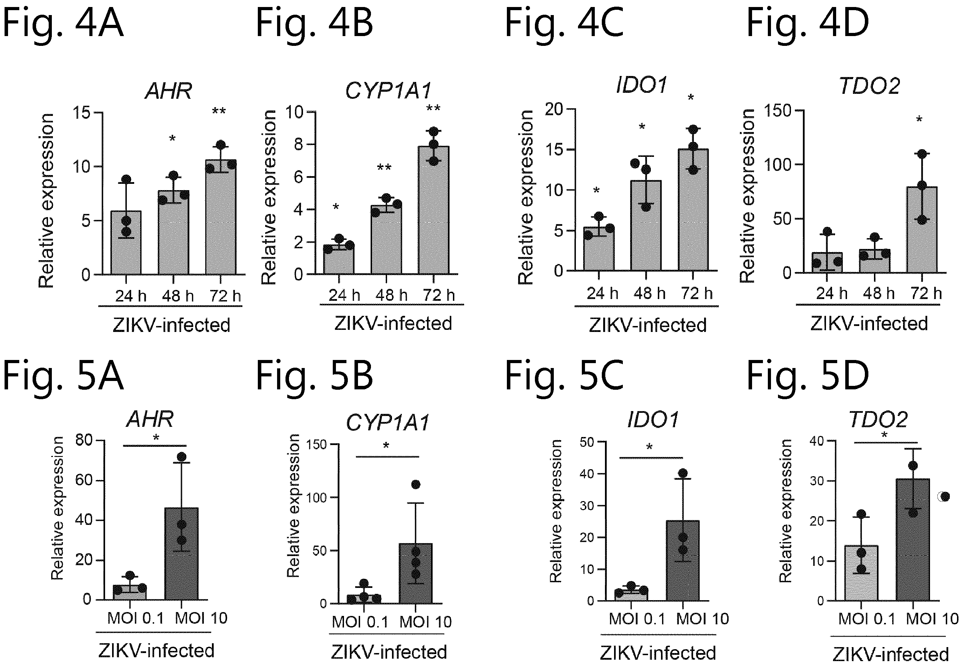

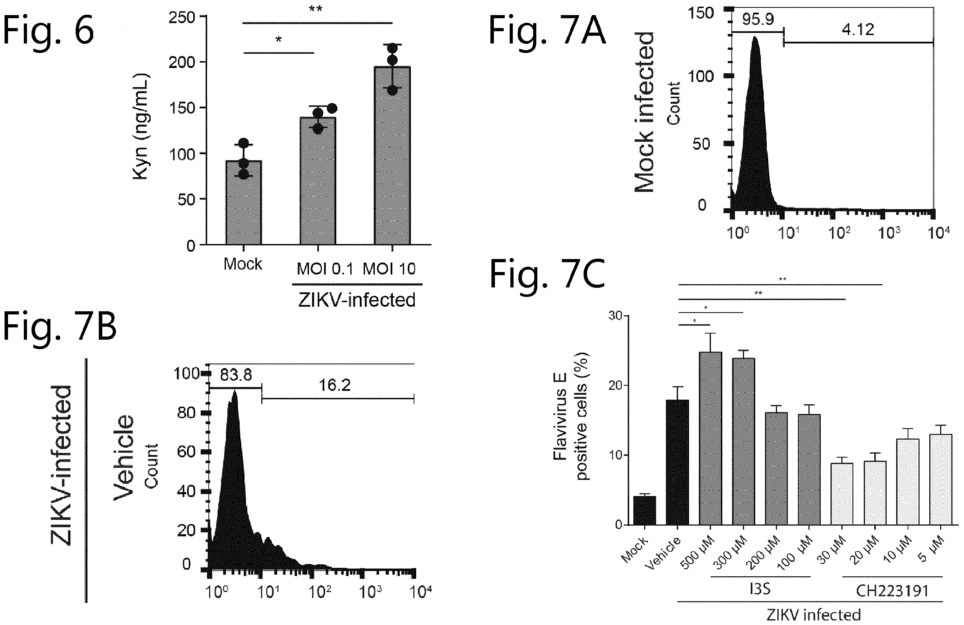

ZIKV replicates in neural progenitor cells (NPCs)²⁴ as part of disease mechanisms thought to promote brain abnormalities in Zika congenital syndrome^{10,25-29}. Indeed, we detected 49% of infection in human primary NPCs exposed to ZIKV. ZIKV infection increased *AHR, CYP1A1, IDO1* and *TDO2* expression in human primary NPCs (Fig. 4). This increase in *AHR, CYP1A1, IDO1* and *TDO2* expression was dependent on the multiplicity of infection (MOI) (Fig. 5), and it was also reflected as an increase in Kyn levels in culture supernatants (Fig. 6). Taken together, these findings show that ZIKV-driven Kyn production activates AHR.

### AHR signaling boosts ZIKV replication in vitro

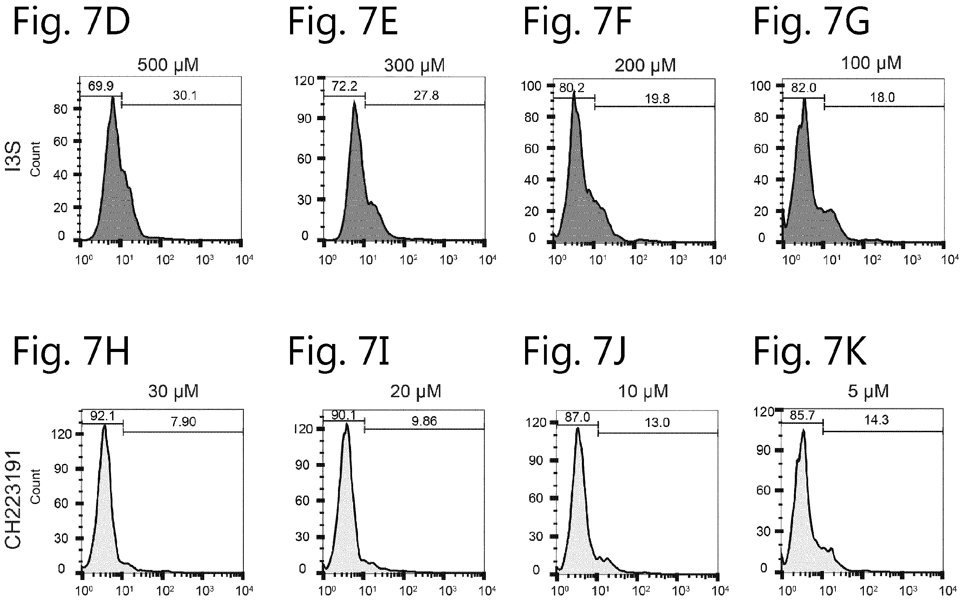

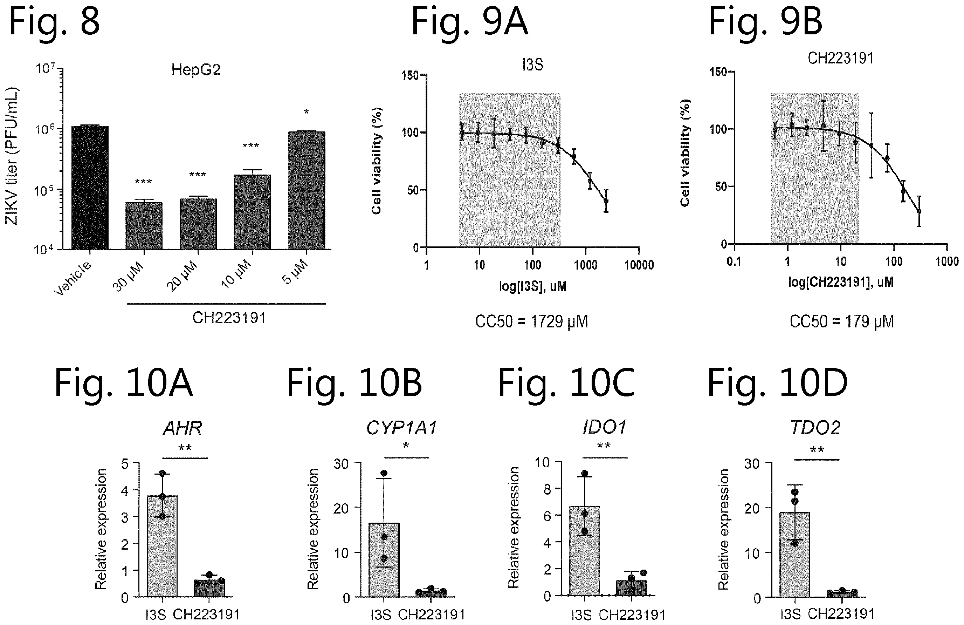

Based on its reported modulatory effects on the immune response and other biological processes³⁰, we hypothesized that AHR signaling may affect ZIKV replication. To investigate the role of AHR on ZIKV infection we used the AHR antagonist CH223191³¹ and the AHR agonist indoxyl-3-sulfate (I3S), a Trp metabolite that reaches the central nervous system (CNS) and is controlled by the commensal flora^{32, 33}. AHR inhibition with CH223191 reduced ZIKV replication (Puerto Rico strain, PRVABC59) by more than 50%, as determined by the quantification of HepG2 cells expressing viral antigen and by the quantification of infective virus particles released in HepG2 cell culture supernatants (Figs. 7 and 8). Conversely, AHR activation with I3S increased the percentage of HepG2 cells expressing viral antigen (Fig. 7). Of note, no cytotoxic effects were detected at concentrations of CH223191 and I3S tested, which resulted in significant AHR inhibition and activation, respectively (Figs. 9 and 10).

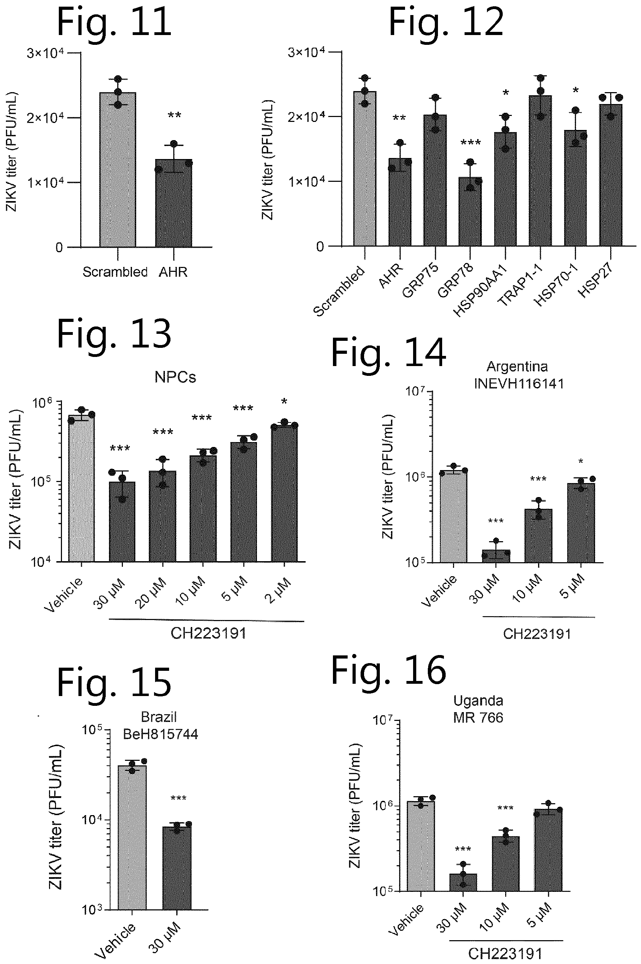

To validate these findings, we knocked down AHR expression in HepG2 cells, detecting a reduction in ZIKV replication that resembled the effects of AHR pharmacological inhibition (Fig. 11). Indeed, the knockdown of AHR led to a reduction of ZIKV yields comparable to that obtained following the knockdown of well-known pro-viral factors such as GRP78³⁴ (Fig. 12). Moreover, AHR inhibition with CH223191 also decreased ZIKV replication in NPCs cultures (Figs. 13), and suppressed the replication of multiple ZIKV isolates from Argentina (INEVH116141), Brazil (BeH815744) and the reference ZIKV strain from Uganda (MR 766) (Figs. 14 - 16).

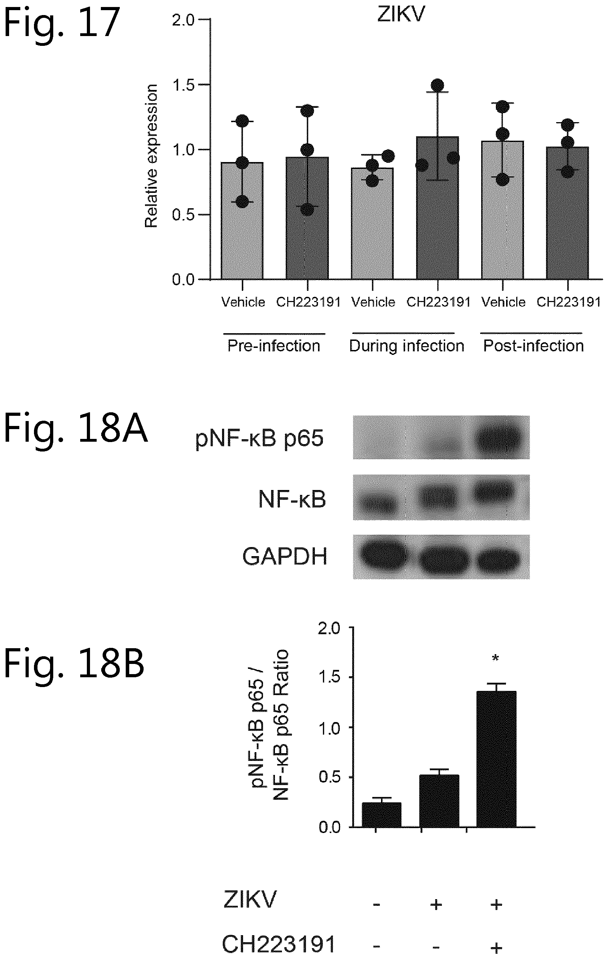

To investigate whether AHR inhibition interferes with the viral entry step, we treated HepG2 cells with CH223191 at different early time points of ZIKV infection (pretreatment before viral entry, during viral adsorption and during virus internalization) and quantified ZIKV by qPCR 2 hours after infection. We detected no effect of CH223191 at these early timepoints during ZIKV infection as determined by qPCR, showing that AHR contributes to later stages of ZIKV replication (Fig. 17). Taken together, these findings show that AHR is a host factor that favors the replication of multiple ZIKV strains in multiple cell types.

### AHR limits IFN-I dependent and PML-driven intrinsic immunity to ZIKV

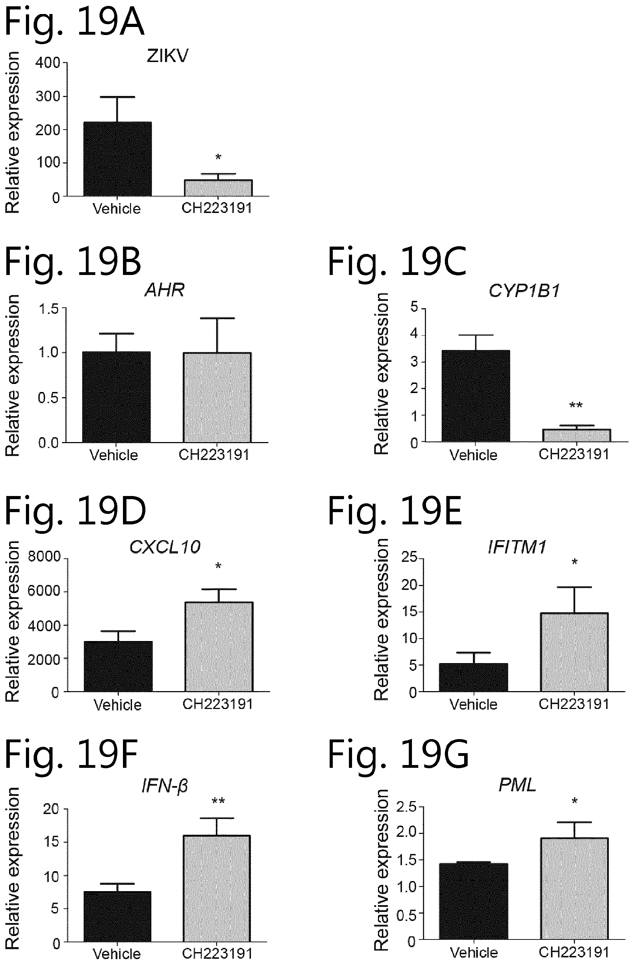

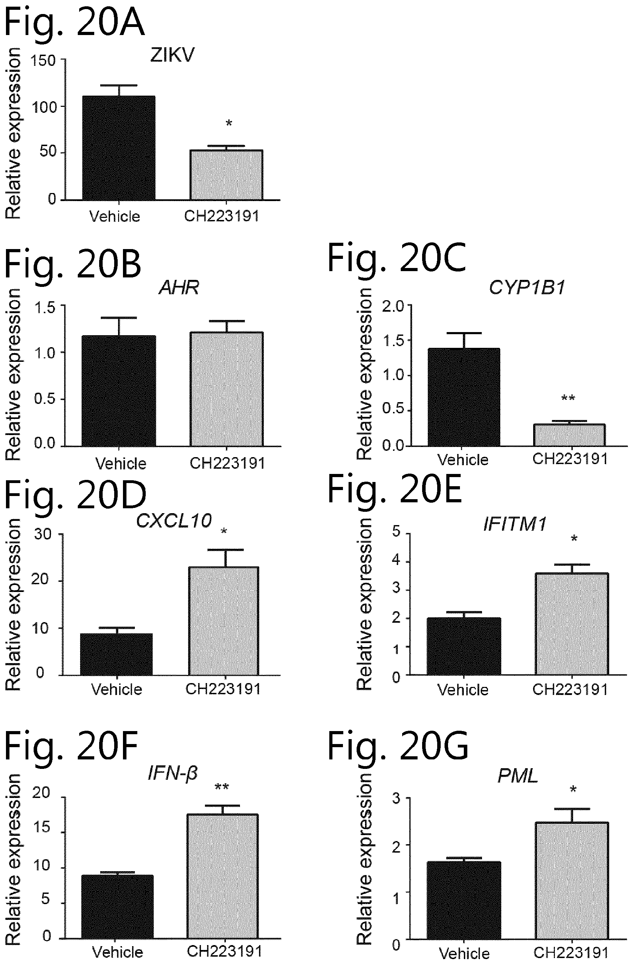

Type I interferons (IFN-I) limit ZIKV replication through interferon-stimulated genes (ISGs)³⁵⁻³⁷. The transcription factor NF-κB drives the expression of multiple antiviral genes, including IFN-I and ISGs³⁸. Since AHR is reported to suppress NF-κB activation^{30, 39} and IFN-I production^{40, 41}, we studied the role of AHR in the control of NF-κB and the anti-viral response during ZIKV replication. ZIKV infection of HepG2 cells triggered a non-significant but detectable increase in NF-κB activation (Fig. 18). AHR inhibition boosted NF-κB activation, IFN-I and ISG expression in ZIKV-infected cells, while it decreased ZIKV RNA levels (Figs. 18 and 19). Similar results were observed following AHR inhibition in human NPCs infected with ZIKV (Fig. 20).

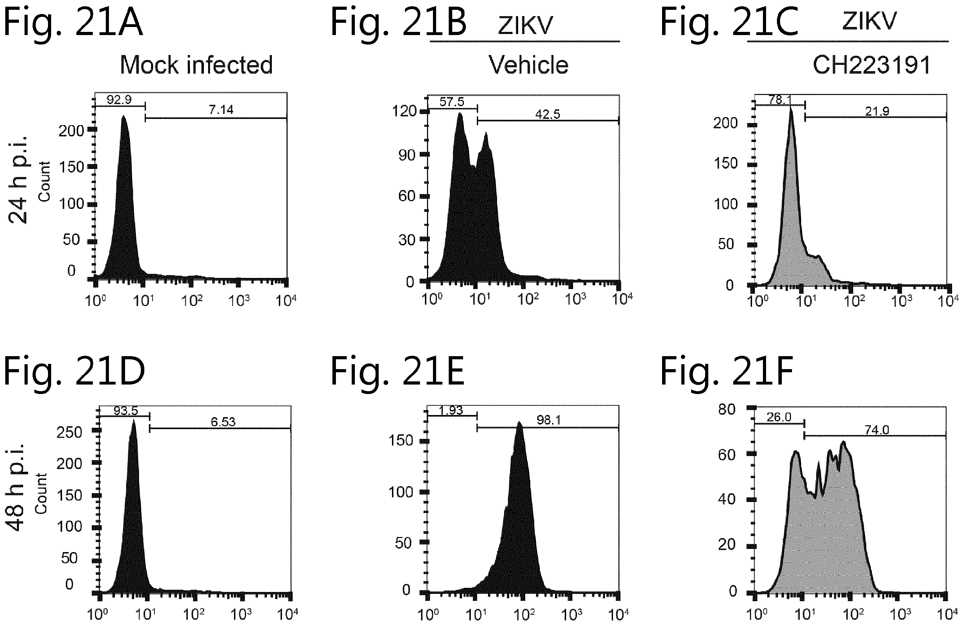

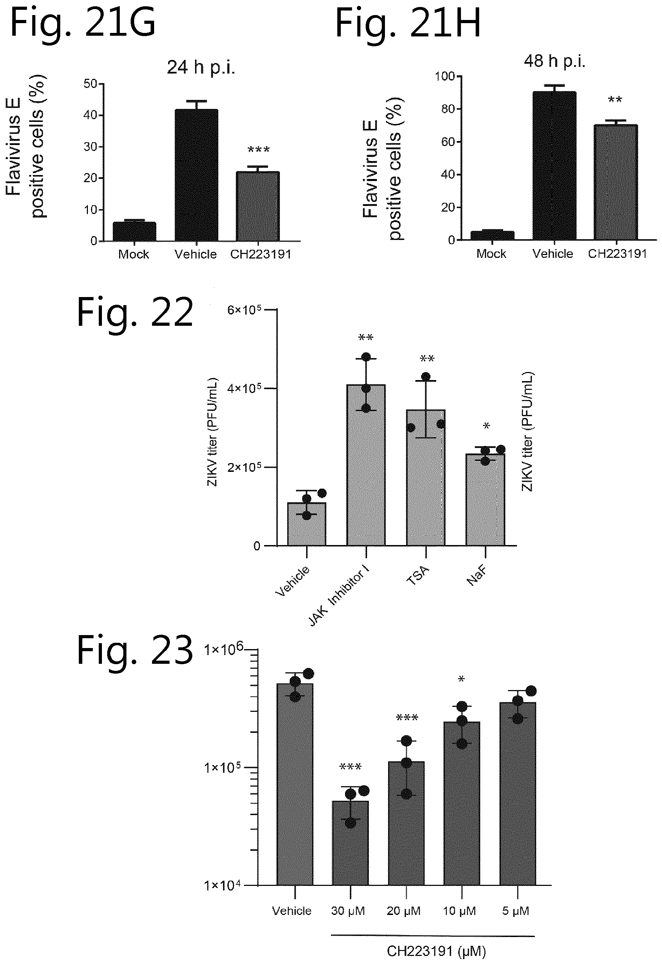

To investigate the contribution of the suppression of IFN-I and ISG expression to the increase in ZIKV replication associated to AHR signaling, we used Vero cells which do not produce IFN-I as a result of spontaneous gene deletions⁴². We detected increased ZIKV replication in Vero cells as indicated by the expression of virus antigen in up to 90% of the cells at 48h post infection (p.i.), compared to 16% achieved in HepG2 cells (Figs. 7 and 21). A similar increase in ZIKV replication was detected when HepG2 cells were infected with ZIKV in the presence of drugs that block IFN-I signaling pathway, such as JAK inhibitor I, trichostatin A and NaF (Fig. 22). Taken together, these findings show that ZIKV-triggered AHR activation suppresses NF-κB driven IFN-I-dependent responses that limit ZIKV replication.

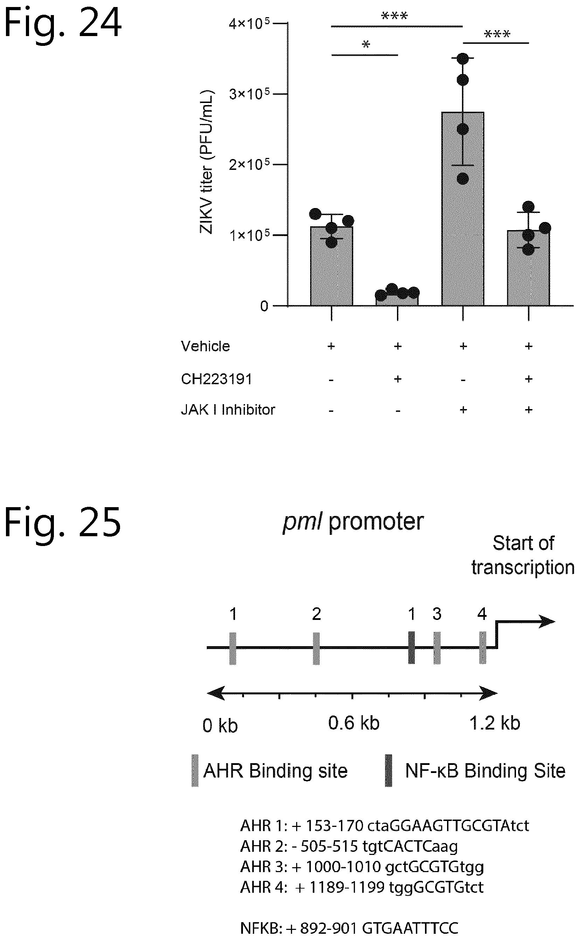

IFN-I independent mechanisms also limit viral replication⁴³⁻⁴⁵. Indeed, AHR inhibition reduced the infection of IFN-I deficient Vero cells by about 30 %; this suppression of ZIKV replication was confirmed by the quantification of virus yield (Figs. 21 - 23). Moreover, AHR inhibition also reduced virus replication when IFN-I signaling was suppressed with a JAK1 inhibitor (Fig. 24). Collectively, these findings show that AHR also interferes with IFN-I-independent anti-viral mechanisms to promote ZIKV replication.

AHR signaling in ZIKV-infected cells suppressed the expression of PML (Figs. 19 and 20), a restriction factor reported to inhibit DENV replication^{16, 46}. PML expression has been suggested to be induced by NF-κB signaling, but the mechanisms involved are still unclear⁴⁷. Based on the ability of AHR to control the expression of target genes directly and through its effects on NF-κB and other transcription factors ^{30, 39}, we investigated the regulation of PML expression by AHR.

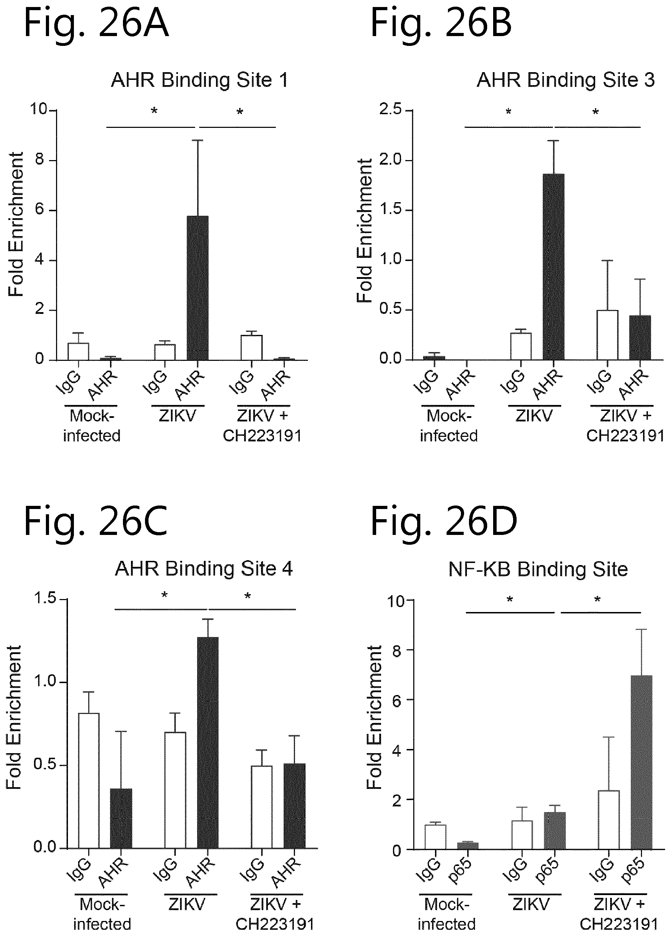

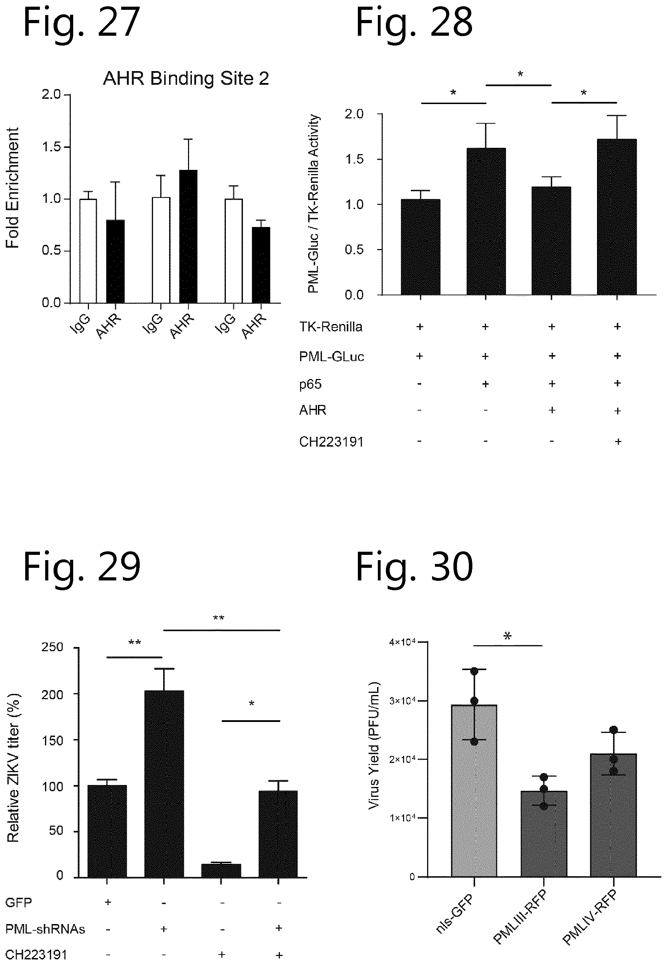

A bioinformatic analysis identified candidate binding sites for AHR and NF-κB in the *Pml* promoter (Fig. 25). In chromatin immunoprecipitation (ChIP) assays, we detected AHR recruitment to its responsive elements following ZIKV infection of HepG2 cells (Figs. 26 and 27). Moreover, NF-κB transactivated the *Pml* promoter in reporter assays, but this transactivation was prevented by the co-expression of AHR (Fig. 28). *Pml* promoter transactivation by NF-κB, however, was rescued by treatment with the AHR antagonist CH223191 (Fig. 28), concomitant with increased recruitment of NF-kB and decreased AHR recruitment to their respective responsive elements (Fig. 26). These data show that AHR limits PML expression by suppressing NF-κB activation as reported in other cell types such as dendritic cells, astrocytes and microglia^{30, 39}, and also, by interacting with AHR-responsive regulatory elements in the *Pml* promoter*.*

To investigate the contribution of PML to the suppressive effects of AHR inhibition on ZIKV replication we performed siRNA knockdown studies. The knockdown of PML expression partially abrogated the suppressive effect of CH223191 on viral replication (Fig. 29). Conversely, the overexpression of PML isoform III, which we recently reported limits the replication of DENV¹⁶, suppressed ZIKV replication (Fig. 30). Collectively, these findings identify PML as a restriction factor for ZIVK replication, and show that AHR limits IFN-I dependent antiviral mechanisms as well as PML-driven intrinsic immunity to ZIKV.

### AHR inhibition reduces ZIKV replication in vivo and ameliorates microcephaly

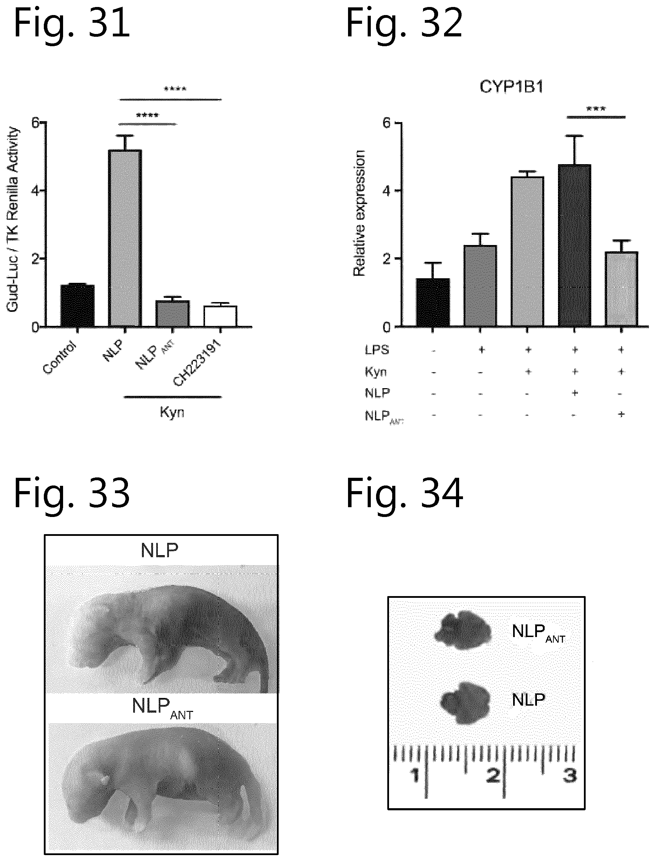

ZIKV infection during pregnancy has been linked to congenital Zika syndrome, characterized by multiple birth defects including microcephaly and/or intra-uterine growth restriction (IUGR)⁸⁻¹⁰. ZIKV infection of pregnant mice of the SJL strain recapitulates several features of congenital Zika syndrome, such as microcephaly and cortical brain damage²⁶. Based on our findings on the role of AHR as a host factor for ZIKV replication, we evaluated the therapeutic potential of AHR inhibition in pregnant SJL mice infected with ZIKV. First, we used the AHR antagonist CH223191 formulated in nanoliposomes (NLP_{ANT}) to increase its solubility and biodistribution; empty nanoliposomes (NLP) were used as controls. NLP_{ANT} inhibited AHR activation by Kyn in a luciferase reporter assay (Fig. 31), and also suppressed the expression of the AHR-driven *Cyp1b1* gene in primary murine dendritic cells (Fig. 32), demonstrating that NLP-formulated CH-223191 can suppress AHR signaling.

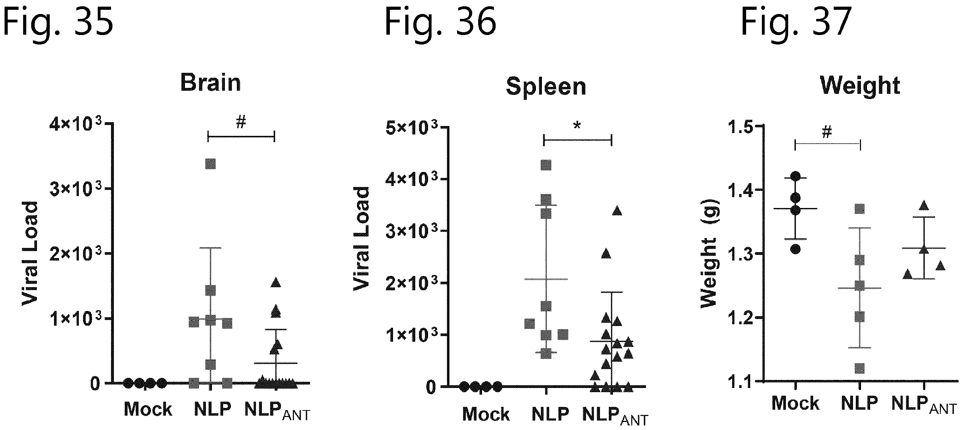

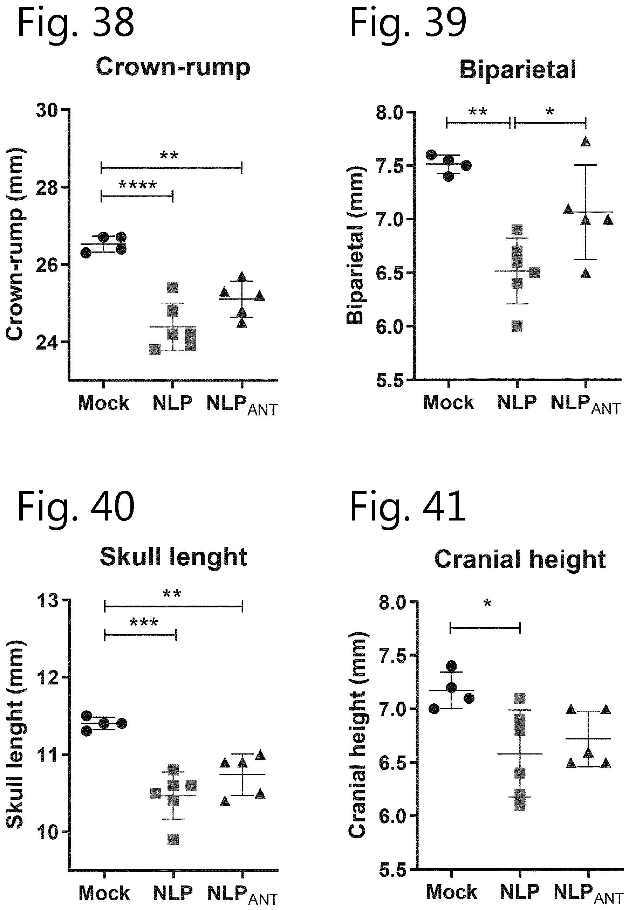

Pregnant female SJL mice were infected with 10⁶ PFUs of ZIKV and treated daily with 100 mg/day of NLP_{ANT} or NLP. NLP_{ANT} treatment ameliorated fetal IUGR and microcephaly (Figs. 33 and 34), while it also reduced ZIKV viral load in brain and spleen (Figs. 35 and 36), indicating that AHR inhibition by NLP_{ANT} limits ZIKV replication *in vivo.* Indeed, NLP_{ANT} treatment reversed the reduction of fetal weight, crown-rump, biparietal, skull length and cranial height induced by ZIKV (Figs. 37 - 41). AHR antagonism by NLP_{ANT} also diminished fetal brain pathology induced by ZIKV, as evidenced by thicker cortical plates and reduced ventricle sizes (Fig, 42).

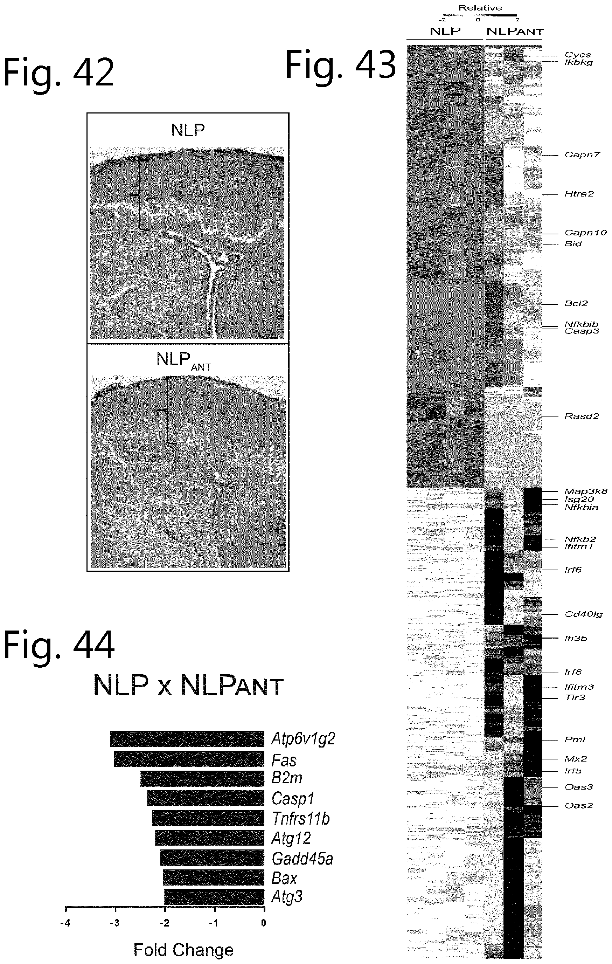

To further investigate the effects of AHR antagonism on f ZIKV-induced defects on fetal development, we analyzed by RNA-seq CNS fetal samples. NLP_{ANT} treatment decreased the expression of genes associated to apoptosis, tissue damage and autophagy, previously linked to ZIKV-induced CNS pathology²⁶ (Fig. 43, table 1).

**Table 1.**

| **Ingenuity Canonical Pathways** | **-log(p-value)** | **z-score** |
|---|---|---|
| Apoptosis signalling | 5 | -0.289 |
| NF-KB activation | 4.74 | 0.469 |
| Induction of apoptosis | 3.82 | -1.061 |
| NF-KB signalling | 3.2 | 1.76 |

Conversely, AHR inhibition with NLP_{ANT} was associated to the increased expression of transcriptional programs indicative of NF-kB activation and IFN-I signaling, and *Pml* upregulation (Fig. 43, table 1). Similar results were obtained when these samples were analyzed using a PCR Array, which detected the upregulated the expression of transcriptional modules associated to NF-κB signaling and anti-microbial immunity following NLP_{ANT} administration. Taken together, these findings show that AHR inhibition boosts transcriptional modules of innate and intrinsic immunity that limit ZIKV replication and induced pathology *in vivo* (Fig. 44, table 2).

**Table 2.**

| **Ingenuity Canonical Pathways** | **P-value** | **Z-score** |
|---|---|---|
| Role of Pattern Recognition Receptors in Recognition of Bacteria and Viruses | 5.01 E-61 | 3.16 |
| TREM1 signalling | 5.01 E-48 | 3.66 |
| Dendritic Cell Maturation | 3.16E-37 | 2.65 |
| Toll-like Receptor signalling | 1.58E-36 | 2.67 |
| NF-KB signalling | 7.94E-26 | 3.71 |
| IL-6 Signalling | 1.26E-18 | 2.67 |
| P38 MAPK Signalling | 7.59E-10 | 3.00 |

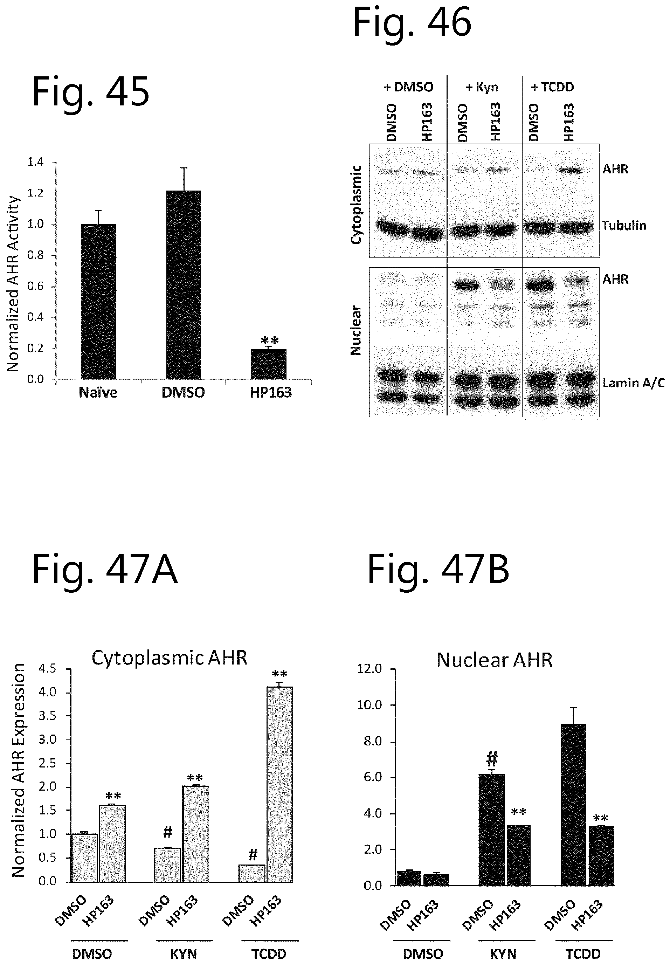

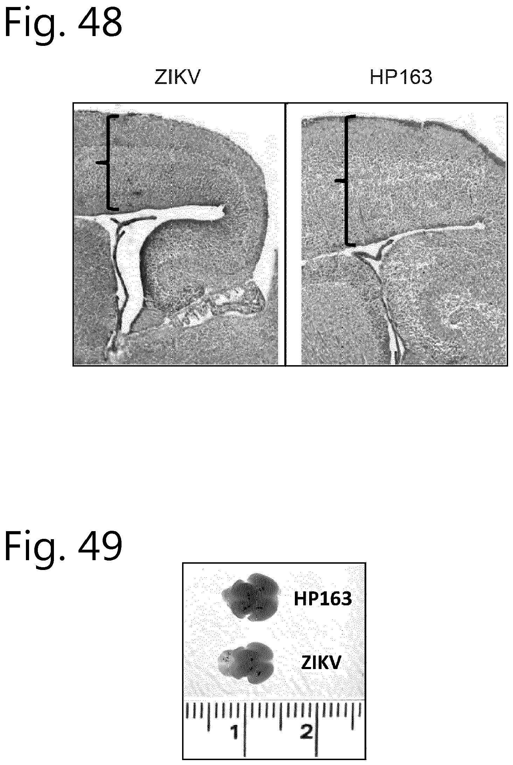

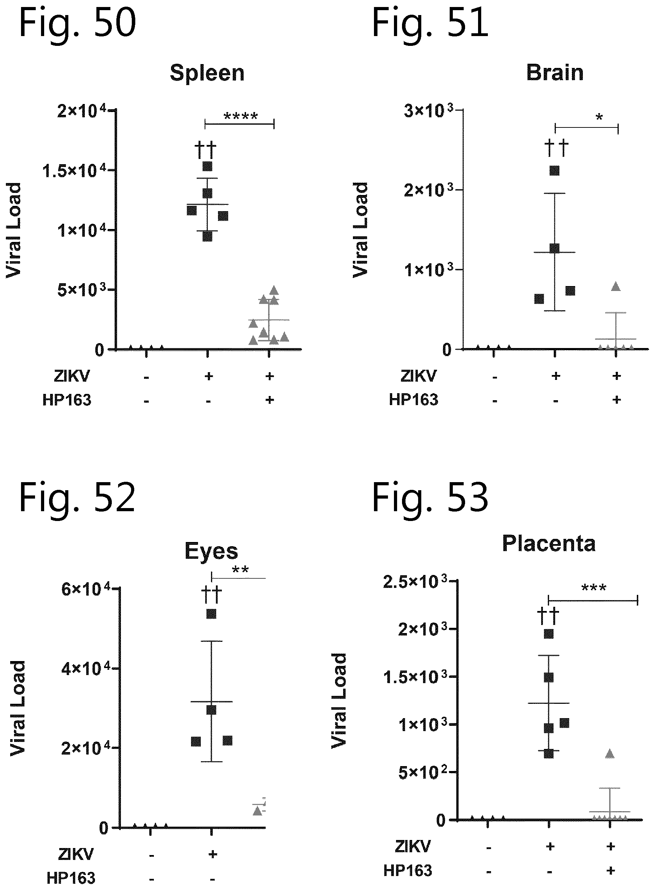

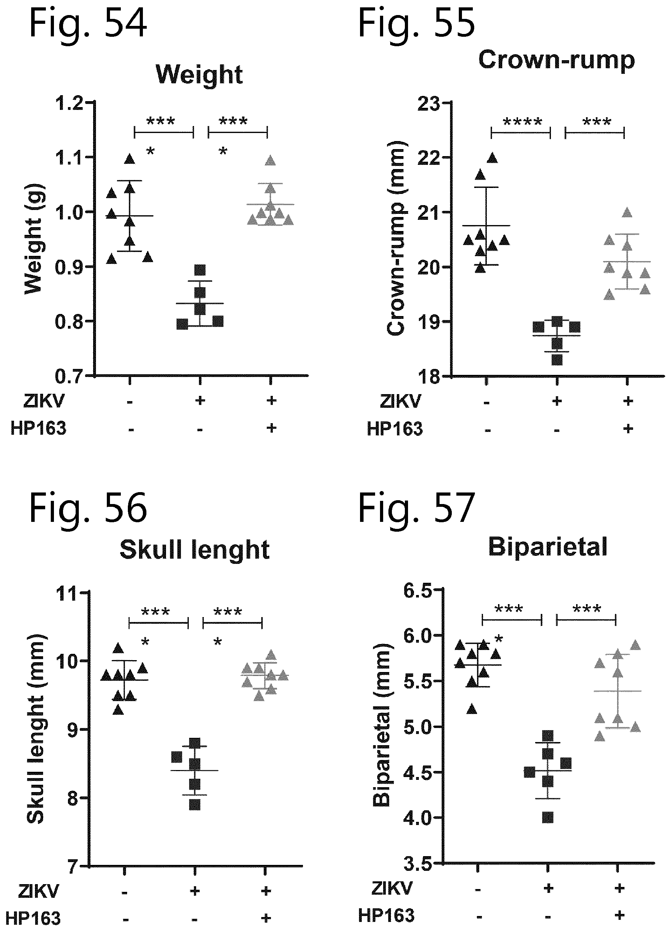

To investigate the translational relevance of AHR inhibition for the management of ZIKV infection, we evaluated HP163, an antagonist developed for the therapeutic inhibition of AHR in cancer, where AHR has been linked to tumor pathology through multiple mechanisms⁴⁸. HP163 suppressed AHR activation as determined in luciferase reporter assays and western blot studies (Figs. 45 - 47). HP163 oral administration to SJL mice reduced CNS pathology associated to ZIKV infection, as indicated by the prevention of ventricular dilation, cortical thinning and overall reduction in brain size (Figs. 48 and 49). Similar to NLP_{ANT}, HP163 reduced fetal ZIVK viral loads in the brain, eyes and spleen (Figs. 50 - 53), and also the viral load detected in the placenta of treated mothers. HP163 administration also suppressed IUGR, as indicated by the preservation of body and skull size and morphometric features (Figs. 54 - 58).

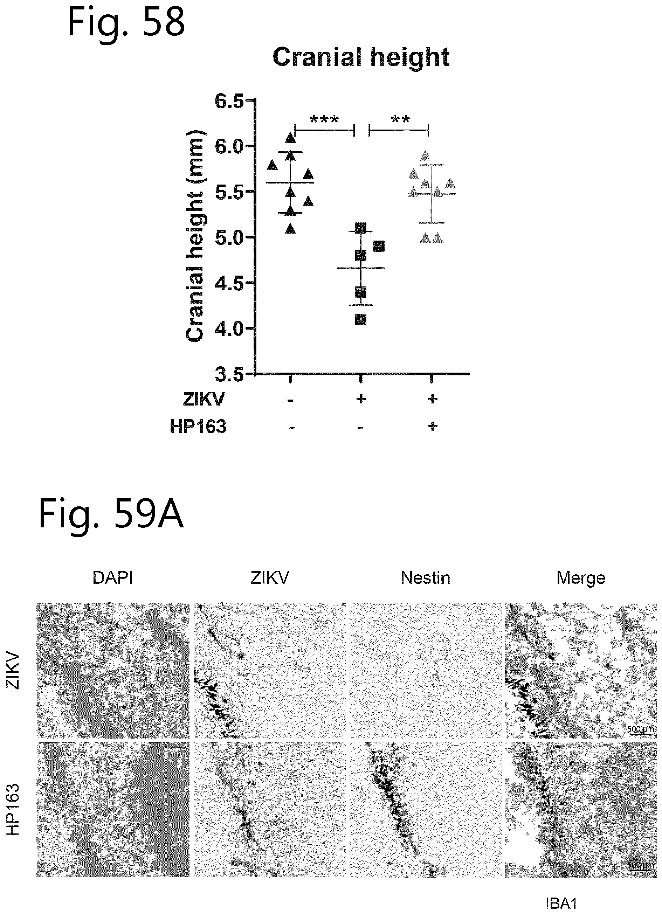

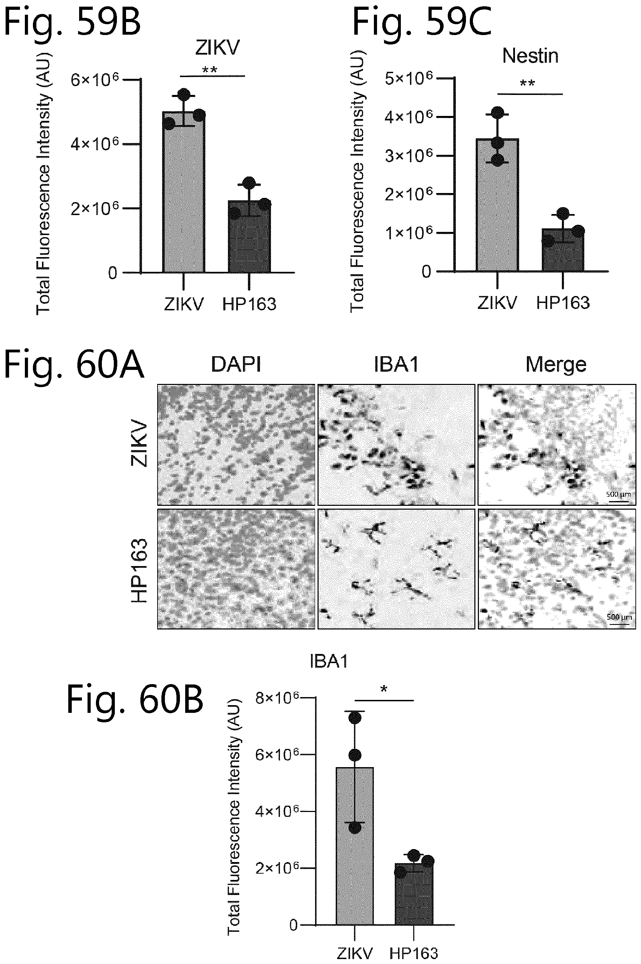

To further evaluate the effects of HP163 *in vivo,* we analyzed by immunofluorescence ZIKV and progenitor cells in the brains of infected fetuses. HP163 administration decreased the number of ZIKV infected hippocampal cells, preserving the number of nestin positive progenitor cells (Fig. 59). In agreement with the reduction of ZIKV titers in the fetal brain, HP163 administration resulted in decreased microglial activation as indicated by decreased *Iba1* expression (Fig. 60). Taken together, these findings show that AHR signalling plays a role in ZIKV replication and pathogenesis *in vivo,* and identify AHR antagonism as a therapeutic approach for congenital Zika syndrome.

### AHR inhibition suppresses DENV replication

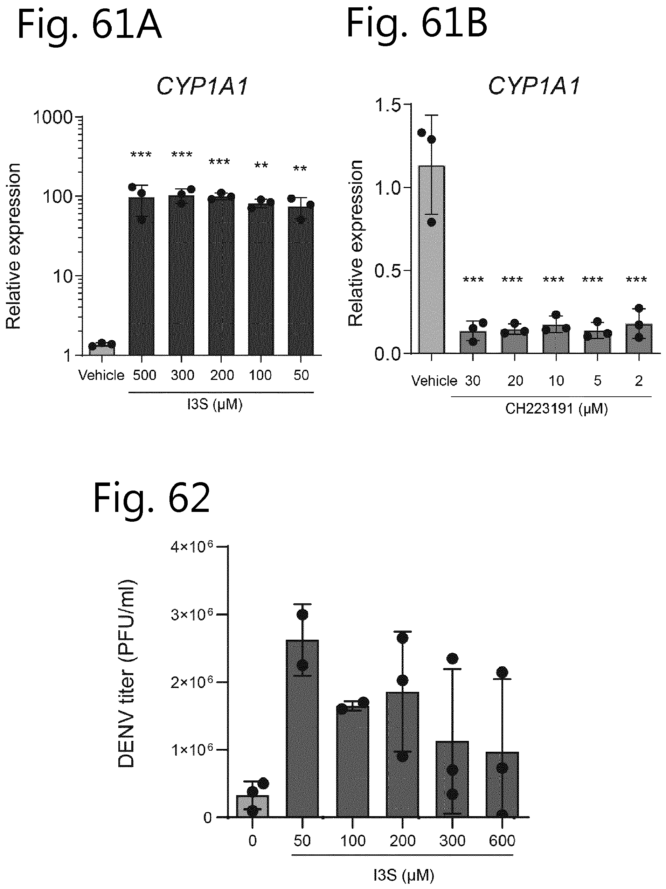

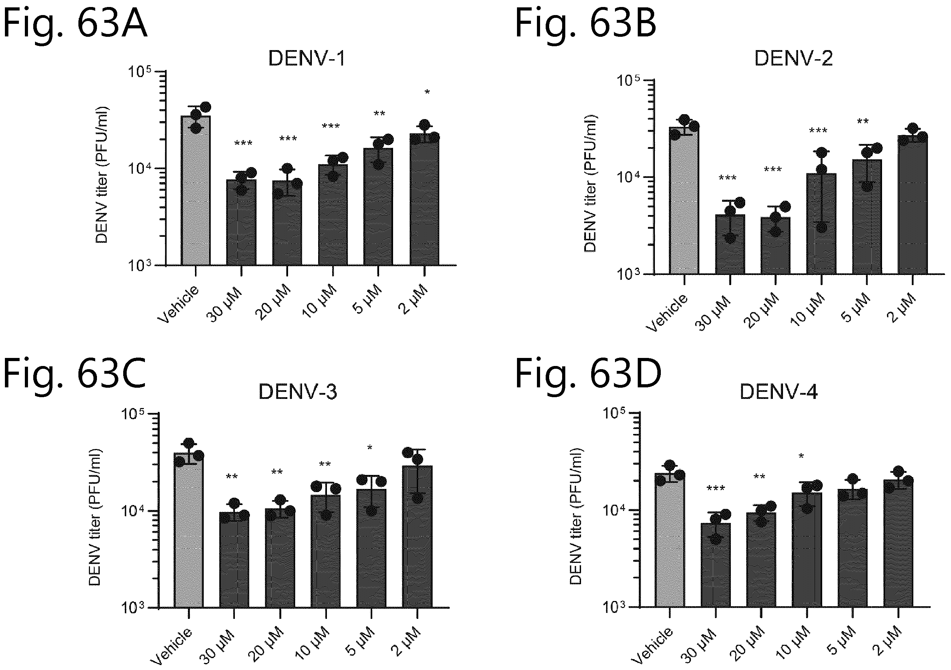

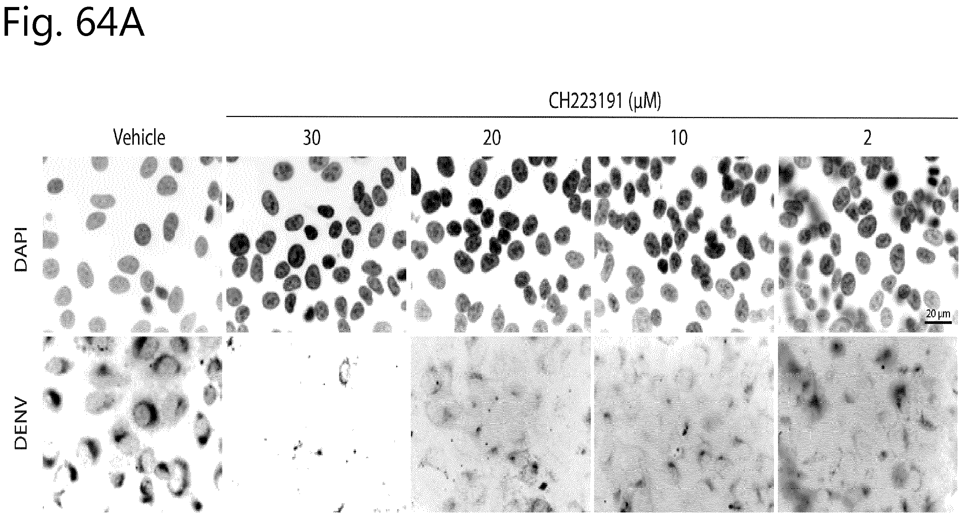

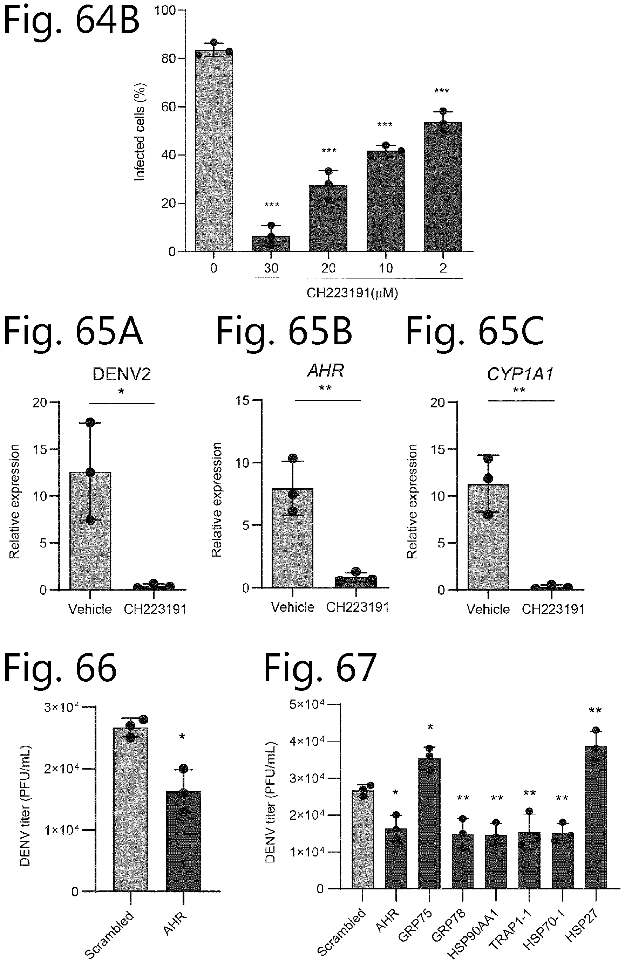

PML and IFN-I limit the replication of the flavivirus DENV^{35, 46}. Based on the suppression of anti-viral responses by AHR and the conservation of host factors supporting flavivirus replication, we investigated the role of AHR in DENV replication. Specifically, we evaluated the effects of AHR modulation on the replication of the four known DENV serotypes in A549 cells. I3S activated AHR in A549 cells and increased the yield of the four DENV serotypes (Figs 61 and 62). Conversely, the AHR antagonist CH223191 reduced the replication of the four DENV serotypes (Fig. 63). This inhibition of DENV production was also detected when viral RNA and viral protein expression levels were quantified (Figs. 64 and 65). Importantly, AHR knockdown significantly reduced DENV yield (Figs. 66) and this inhibition was comparable to the reduced extracellular viral production achieved by the knockdown of other targets reported to promote DENV replication such as GRP78 and HSP70^{34, 49} (Fig. 67). Taken together, these findings show that AHR also promotes DENV replication.

We identified AHR as a host factor for ZIKV replication. ZIKV-triggered AHR activation suppresses IFN-I expression (Scheme 1).

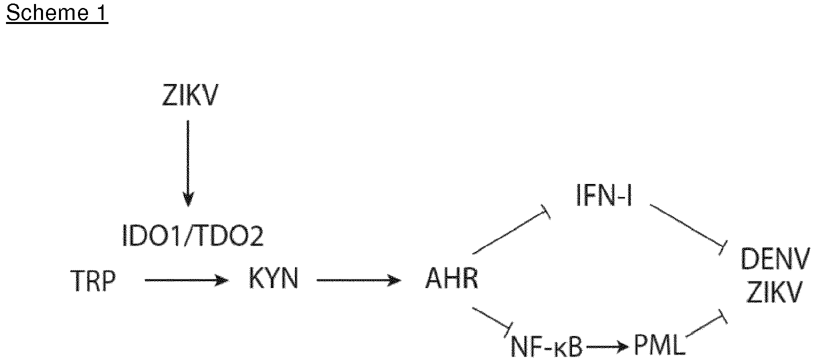

Of note, although IFN-I plays a central role in anti-viral immunity, dysregulated IFN-I signaling mediates detrimental inflammation and pathology induced by viral infections^{55, 56}. Based on the upregulation of AHR expression by IFN-I³³, IL-6⁵⁷, IL-27^{58,59} and other cytokines involved in antiviral immunity, these findings show that AHR participates in a negative feedback regulatory loop that limits immunopathology. Hence, these findings show that ZIKV exploits AHR-driven immunoregulatory mechanisms to evade the immune response.

ZIKV infection may also induce the production of additional agonists besides Kyn and/or suppress the degradation of natural agonists by inhibiting cytochrome P4501 (CYP1) enzymes⁶² to further limit IFN-I dependent and IFN-I independent anti-viral mechanisms via AHR.

Using a murine model of ZIKV infection, we found that AHR inhibition limits viral replication, identifying AHR antagonists are potential therapeutics for ZIKV infection. AHR signaling also modulates anti-viral adaptive immunity⁶³⁻⁶⁶. Hence, AHR antagonists not only limit ZIKV replication and congenital Zika syndrome, they also boost the activity of ZIKV vaccines currently under development⁶⁷⁻⁶⁹. Based on the role of AHR in the control of multiple aspects of anti-viral immunity, our work supports repurposing AHR antagonists developed for cancer immunotherapy⁷⁰⁻⁷² for the management of ZIKV and potentially, DENV infection. The available pre-clinical data show that AHR inhibition with HP163 does not result in overt toxicity.

PML is reported to limit viral replication at multiple levels, sequestering viral proteins, inhibiting viral mRNA synthesis and inducing apoptosis and autophagy in infected cells^{46, 67-69, 73-75}, but its role in ZIKV infection was previously unknown. Moreover, little is known about IFN-independent signaling pathways that control PML expression⁷⁴. We identified PML as a factor that limits ZIKV replication in an IFN-I independent manner (Scheme 1). In addition, we identified NF-κB as a driver of PML expression, and we also found that AHR interferes with *PML* expression by limiting NF-KB activation. However, AHR controls phosphorylation cascades²² and ubiquitin-driven protein degradation⁷⁶, and post-translational modifications regulate PML activity⁷⁴. Hence, it is conceivable that ZIKV-driven AHR signaling interferes with PML-driven intrinsic antiviral immunity at multiple levels. Nevertheless, these findings show that the balance between NF-κB and AHR signaling regulates PML-driven intrinsic immunity to ZIKV, and potentially other viruses such as DENV^{16, 46}.

Individuals within a population show heterogeneous susceptibility to viral infections and virus-induced pathology, but the mechanisms involved are poorly understood. Environmental factors such as pollutants, the diet and the microbiome affect the course and clinical outcome of infections by multiple viruses, including ZIKV ^{77, 78}. AHR agonists are not only produced by host metabolism, they are also provided by environmental pollutants, the diet and the commensal flora³⁰. Indeed, microbial metabolites derived from dietary Trp activate AHR in astrocytes and microglia, limiting inflammation and neurodegeneration^{32, 33}; microbial metabolites also activate AHR in the liver⁷⁹. Considering the role of the liver and the CNS in ZIKV replication and pathology, our findings show that the modulation of AHR signaling by environmental agonists affects multiple aspects of ZIKV infection.

In summary, we identified AHR as a ZIKV host factor, which interferes with IFN-I driven and PML-dependent immunity (scheme 1). These studies identify AHR as a target for the treatment, amelioration and/or prevention of ZIKV and other flaviviruses. In addition, our findings define AHR signaling as a pathway by which environmental factors influence the course of ZIKV infection and its associated pathology.

Hence, the invention relates to an antagonist of the aryl hydrocarbon receptor (AHR) for use in the treatment, amelioration or prevention of congenital ZIKA Virus (ZIKV) syndrome. Antagonists of the AHR are well known in the field and a skilled person will have no difficulties to find existing AHR antagonists or develop them using known methods and assays. As an example of the variety of AHR antagonists that have been described to date, the following structural formulas are provided.

| **Name** | **Structure** |
|---|---|
| CAS 301326-22-7 | 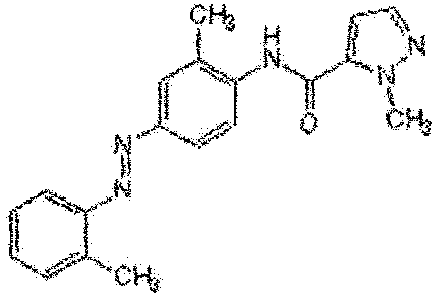 |
| GNF351 | 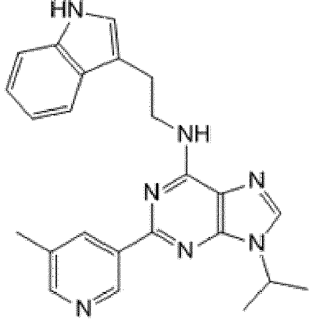 |
| CAS 2162982-11-6 | 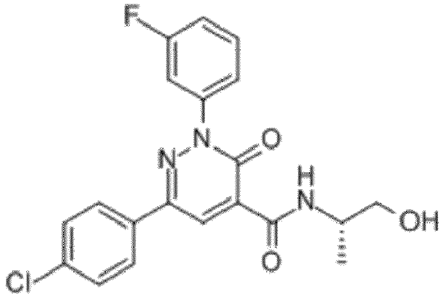 |
| Stemreginin 1 | 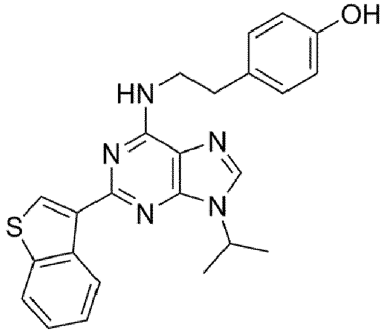 |
| 31 | 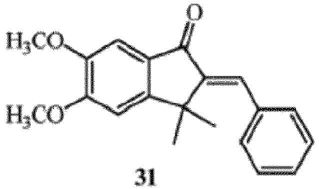 |

In US 10,314,810 B2 (which is incorporated herein by reference) the following general formula is provided as having AHR antagonist activity:

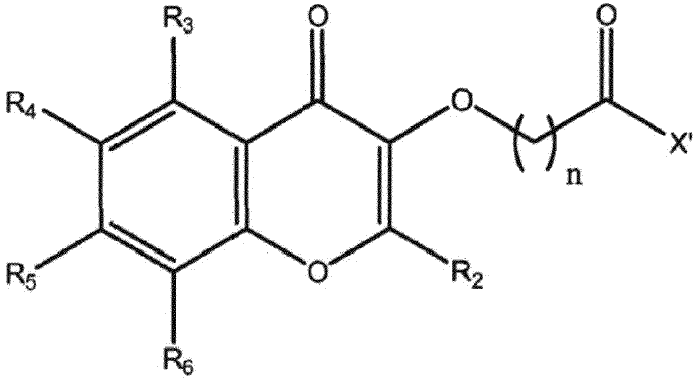

Formula 1, general formula for AhR antagonists, wherein:
X' is H, unsubstituted alkyl, aminosulfonyl, alkoxy, amino, acyl, aryl, or heteroaryl, each of which may be optionally substituted;
n is 0-6;
R2 is H, alkyl, acyl, heteroaryl, arylalkyl, cycloalkyl, heteroarylalkyl, heterocyclyl, or haloalkyl, each of which may be optionally substituted,
R3, R4, R5 and R6 are independently H, alkyl, acyl, halo, aryl, or heteroaryl, each of which may be optionally substituted, or
a stereoisomer or
a pharmaceutically acceptable salt thereof.

In Smith et al., J. Pharmacology and Exp. Therapeutics (2011) 338(1):318-327, the following structures are disclosed as having AHR antagonist activity. 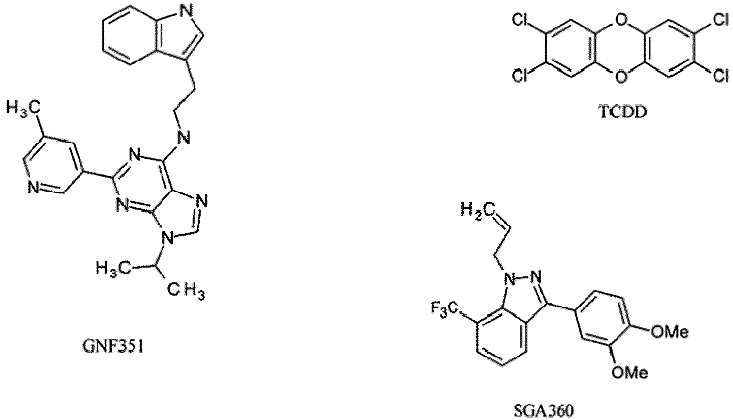 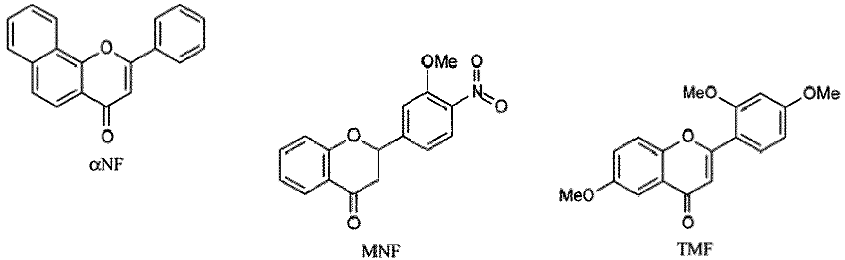 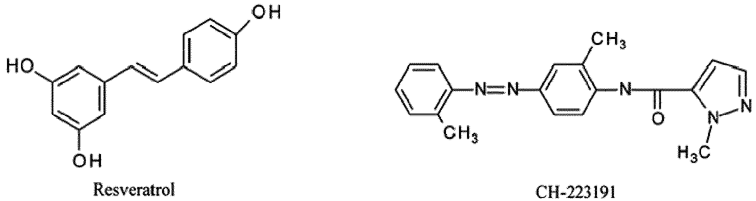 In Parks et al., Molecular pharmacology (2014) 86(5) 593-608, the following structures are disclosed as AHR antagonists.

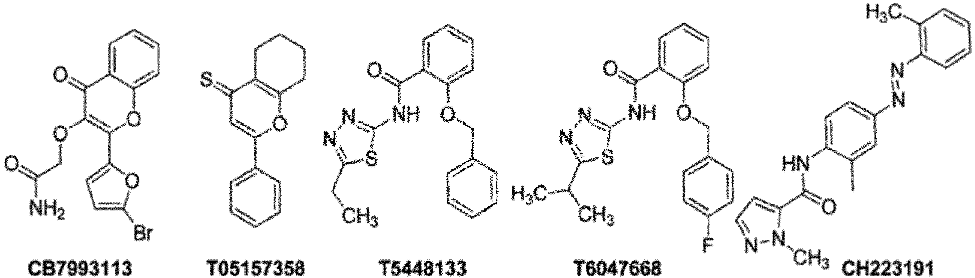

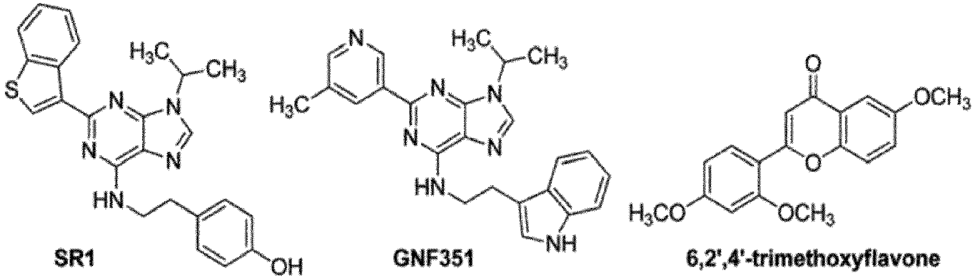

### Examples

### Example 1; methods, cells, antibodies and reagents.

HepG2 (ATCC, HB-8065), A549 (ATCC® CCL-185™), HeLa (ATCC, CCL-2) and Vero cells (African green monkey kidney) (ATCC, CCL-81) were grown in Dulbecco's Modified Eagle's medium (DMEM, GIBCO) supplemented with 10 % fetal bovine serum, 100 IU/ml of penicillin and 100ug/ml of streptomycin. HEK-293 cells (ATCC, CRL-1573) and SUM149 cells (Asterand Bioscience, Detroit, MI) were grown in DMEM/F12 (GIBCO) supplemented with 10 % fetal bovine serum, 100 IU/ml of penicillin and 100ug/ml of streptomycin. C6/36 mosquito cells from *Aedes albopictus* were used to generate viral stocks, and were cultured in L-15 medium (Leibovitz) (GIBCO) supplemented with 0.3 % tryptose phosphate broth, 0.02 % glutamine, 1 % MEM non-essential amino acids solution and 10 % fetal bovine serum. Human NPCs (Stem Cell Technologies, Catalog # 70901) were grown using neural progenitor medium 2 (Stem Cell Technologies). Murine DCs were generated from bone marrow cells isolated from the femurs and tibiae of naïve B6 mice and cultured for 9 days in the presence of granulocyte macrophage colony-stimulating factor (GM-CSF, 20 ng/ml) in not treated Petri dishes. Cells were collected at day 9 and BMDCs were characterized as CD11c+MHC-II+Ly6G- cells.

Antibodies were used as follows: anti-flavivirus (Santa Cruz Biotechnology, 1:200 for flow cytometry), anti-AHR (Abcam ab2769, 5 µg per ChIP reaction), anti-NF-κB p65 (Cell Signaling, #D14E12, 1:1000 for WB and 5 µg per ChIP reaction), anti-GAPDH (Cell Signaling, #14C10, 1:1000 for WB), anti-phospho-NF-κB p65 (Ser536) (Cell Signaling, #93H1, 1:1000 for WB), anti-rabbit IgG HRP-linked (Cell Signaling, #7074, 1:3000 for WB).

### Example 2: Small molecules.

I3S (Indoxyl 3-sulfate, Sigma, used at 50-500 µM as indicated for each experiment), CH223191 (Tocris, used at 2-30 µM), Sodium fluoride (Sigma, 1 mM), Trichostatin A (Sigma, 100 nM), JAK I Inhibitor (Santa Cruz Biotechnology, 1 µM). HP163 was the generous gift of Hercules Pharmaceuticals, BV, (Galileiweg 8, 2333 BD, The Netherlands). CB7993113 has been described previously⁸⁰. HP163, is a monoalkylated amide of CB7993113 optimally formulated in a 5% tween, 45% PEG, 50% saline mixture. As tested by Pharmacelsus, GmbH, a PK/PD contract research organization, the maximal plasma concentration of HP163 after i.v. administration of 5 mg/ml in mice is 4.1 ug/ml and its plasma half-life is 3.4 hours. After oral gavage, 51% of the drug is bioavailable and 25% penetrates the blood brain barrier. Dose escalation studies show no acute HP163-mediated toxicity at least up to doses of 300 mg/kg in mice, rats, and dogs; no toxicity was seen in mice after daily dosing with 100 mg/kg over a six months period. HP163 is negative in the hERG (K+ channel patch-clamp) cardiotoxicity assay. A CEREP diversity panel evaluated by Eurofins Discovery demonstrated no reactivity in 70 binding assays for G-coupled protein receptors, transporters, ion channels or nuclear receptors and in 27 enzyme assays including kinases and metabolizing enzymes. The structural formulas of the compounds used in this study are reproduced below.

Formulas:

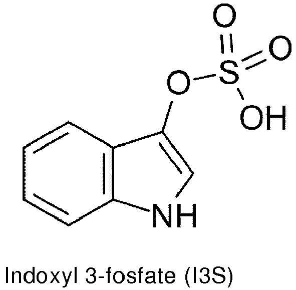

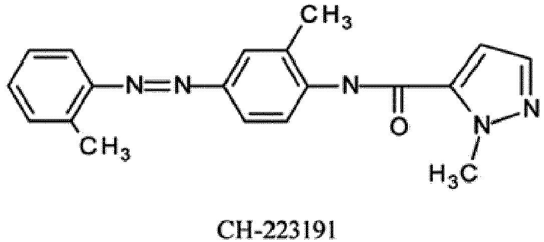

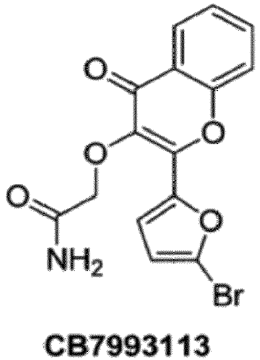

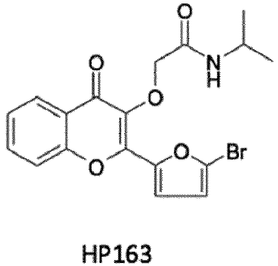

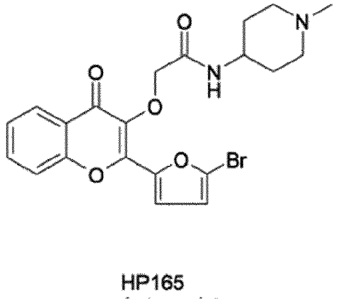

### Example 3: AHR ligands and treatments.

Stock solutions of I3S and CH223191 were prepared in DMSO, aliquoted and stored according to manufacturer's instructions. For CH223191, cells were pre-treated for 90 minutes and then used for further studies. For I3S, cells were pre-treated overnight.

### Example 4: Cell viability assay.

Cell cultures grown in 96-well plates were exposed for 48 h to serial twofold compound dilutions, three wells for each concentration and viability was measured with 3-(4,5-dimethylthiazol-2-yl)-2,5-diphenyl tetrazolium bromide (MTT, Sigma-Aldrich).

**Example 5: Viral stocks.** C6/36 mosquito cells were infected at a multiplicity of infection of 1 with ZIKV Puerto Rico strain (PRVABC59, ATCC isolate gift from MIT laboratory); viral isolate from Argentina (INEVH116141) provided by Instituto Nacional de Enfermedades Virales Humanas, Pergamino, Argentina, ZIKV Brazil (BeH815744) gene bank accession number KU365780.1 gently provided by Prof. Jose Luiz Proença Modena and Daniele Durigon and the reference ZIKV strain from Uganda (MR766), respectively, for 1 h at 37 °C in serum-free DMEM. Quantification of viral titers was performed by a standard plaque assay in Vero cells.

### Example 6: Flow cytometry staining and acquisition.

Cells were fixed and permeabilized using Cytofix/Cytoperm (BD Biosciences). Secondary antibody for flow cytometry was phycoerythrin (PE) goat anti-mouse IgG (BD Biosciences) at a concentration of 1:200. Cells were analyzed on a Guava easyCyte flow cytometer and data was processed using FlowJo software.

### Example 7: qPCR.

RNA was extracted using the RNAeasy kit (Qiagen). cDNA was synthesized using the High-Capacity cDNA Reverse Transcription kit (Applied Biosystems) and qPCR was performed using TaqMan Fast Advanced Master Mix (Applied Biosystems). Gene expression was normalized to GAPDH. Primers and probes (Applied Biosystems) were: *GAPDH* Hs02786624_g1, *IFNB1* Hs01077958_s1, *AHR* Hs00169233_m1, *PML* Hs00971694_m1, *CYP1A1* Hs01054796_g1, *IFITM1* Hs00705137_s1, *CYP181* Hs02382916_s1, *CXCL10* Hs00171042_m1, *IDO1* Hs00984148_m1, *IDO2* Hs01589373_m1, *IFITM Hs00705137_s1, TDO* Hs00194611_m1. Primers and probes for the specific detection of Zika virus RNA were utilized as previously described^{81, 82}. For viral load determination *in vivo,* brains and spleens from pups were macerated on trizol (Invitrogen) for RNA extraction. RNA pellets were resuspended in RNase-free water and quantified by spectrophotometry (NanoDrop -Thermo Scientific) and stored at -80 °C. Primers/probes specific for ZIKV were from Sigma Life Science and sequences ZIKV 835, ZIKV 911c and ZIKV 860-FAM were used as previously described⁸³. qPCR was performed with 10 µl of each sample and 10 µl of the AgPath-IDTM One-Step RT-PCR reagents (Applied Biosystems). The amplification was performed using Quanti Studio 3 thermo cycler (Thermo Scientific). Beta-actin was used as endogenous control of the reaction. Quantification was compared with threshold cycle (Ct) value with a ZIKV plasmid standard curve.

### Example 8: Plasmids, transfections and luciferase assays.

PML shRNA plasmid (sc-36284-SH) was obtained from Santa Cruz Biotechnology. Transfection of HeLa cells was performed using Lipofectamine 2000 (Thermo Fisher) according to manufacturer's instructions. The plasmid encoding for the promoter region of PML, placed upstream of the Gaussia Luciferase (GLuc) reporter gene, was obtained from Genecopoeia. For the luciferase assay, HEK-293 cells were grown in 96 well-plates and transfected using Fugene-HD Transfection Reagent (Roche). Plasmids used for transfection in each condition are indicated in the experiment. 24 h later, pGudluc activity was analyzed using BioLux Gaussia Luciferase Assay Kit (New England Biolabs) and normalized to TK-Renilla luciferase activity, measured using the Renilla Luciferase Assay System (Promega).

### Example 9: siRNA-mediated knockdown.

HepG2 cells were transfected with 50 nM siRNA targeting AHR (Origene, SR319302), HSPA9 (Qiagen FlexiTube GeneSolution GS3313, cat. No. GS3313), HSPA5 (Qiagen FlexiTube GeneSolution GS3309, cat. No. GS3309), HSP90AA1 (Qiagen FlexiTube GeneSolution GS3313, cat. No. SI00075971), TRAP1 (Qiagen FlexiTube GeneSolution, cat. No. SI00115150), HSPA1A (Qiagen FlexiTube GeneSolution GS3303, cat. No. GS3303), HSPB2 (Qiagen FlexiTube GeneSolution GS3316, cat. No. GS3316) or non-targeting scrambled siRNA (Santa Cruz Biotechnology, sc-37007). Briefly, a mix containing 25 pmol siRNA and 1 µl Lipofectamine 2000 was prepared in 100 µl of Opti-MEM (Thermo Fisher Scientific), incubated at room temperature for 20 min and then added to the cells. 48 h post transfection, cells were used for downstream applications (viral infection and plaque assay).

### Example 10: In silico PML Promoter analysis.

PML genomic sequence was obtained from NCBI. The DNA sequence of 1200 bp upstream of the protein coding regions was analyzed. P65/ReIA and AHR binding sites were identified using Mulan⁸⁴.

### Example 11: Chromatin immunoprecipitation (ChIP).

HepG2 cells were mock-infected or infected with ZIKV. 48 h p.i. protein-DNA complexes were cross-linked using 1% paraformaldehyde and lysed with 350 µl lysis buffer (1% SDS, 10 mM EDTA, 50 mM Tris-HCI, pH 8.1 and 1× protease inhibitor cocktail). Chromatin was sheared by sonication, supernatants were collected after centrifugation and diluted in ChIP incubation buffer (1% Triton X-100, 2 mM EDTA, 150 mM NaCl, 20 mM Tris-HCI, pH 8). 5 µg of antibody (anti-AHR or anti- NF-κB p65) was prebound to protein A- and protein G-Dynal magnetic beads (Invitrogen, USA) for 6 h with rotation at 4 °C, washed three times with ice-cold 1% BSA in PBS, and finally added to the diluted chromatin. After an overnight incubation, the magnetic bead-chromatin complexes were washed three times with RIPA buffer followed by two times with TE buffer. Immunoprecipitated chromatin was then incubated with crosslink reversal buffer (1% SDS, 0.1 M NaHCO3) and heated at 65 °C for 6 h. DNA fragments were purified with a QIAquick DNA purification kit (Qiagen, USA) and analyzed using the SYBR Green real-time PCR kit (Takara Bio Inc., USA). The following primer pairs were used: AHR Binding site 1 forward, 5'-CGTAAGTCAGCGGTAGGTCTG -3' (SEQ ID NO: 1), and reverse, 5'-AGAGGCCGACTGTGGGTTTT -3' (SEQ ID NO: 2); AHR Binding site 2, 5'-CAGCTGTGGGCTCTCCTTTC -3' (SEQ ID NO: 3), and reverse, 5'-TACGGTAAAGCGGGAGAGGTA-3' (SEQ ID NO: 4); AHR Binding site 3 forward, 5'-TGACATGCTTTTCCATTGGCG -3' (SEQ ID NO: 5) and reverse, 5'-AGCAGGATGATGCCTTGAGT-3' (SEQ ID NO: 6); AHR Binding site 4 forward, 5'-AGATGGCTGCCCACCATATTT-3' (SEQ ID NO: 7), and reverse, 5'-TGATAGGGCCTGGCTCTTGTA-3' (SEQ ID NO: 8); NPκB Binding site forward, 5'-CCCACCACCTACAACCCTAAA-3' (SEQ ID NO: 9), and reverse, 5'-CATTCTGGAAAGGAGAGCCC -3' (SEQ ID NO: 10).

### Example 12: Measurement of Kyn and Trp concentrations by ELISA.

Kyn and Trp were quantified by ELISA (K3728 and K3730, ImmunDiagnostik) following manufacturer's instructions.

### Example 13: Immunoblot analysis.

Cells were lysed with Cell Lysis buffer (1X) supplemented with protease inhibitor cocktail (Cell Signaling, USA). Total cell lysates (5-10 µg) were resolved on 4-12% Bis-Tris Nupage gels (Invitrogen, USA) and transferred onto PVDF membranes (Millipore) and developed with antibodies for GAPDH, NF-κB p65, Phospho-NF-κB p65, and anti-Rabbit IgG HRP-linked antibody using SuperSignal West Femto Maximum Sensitivity kit (Thermo Scientific), Data quantification was done using Image J software 1.48v (NIH).

### Example 14: RNA-sequencing. RNA was extracted using the RNAeasy kit (Qiagen).

3' DGE RNA-sequencing and library preparation were performed by The Broad Institute of Harvard and MIT. RNA-seq libraries were prepared according to the single cell RNA-seq libraries⁸⁵. Reads were aligned using BWA Aln version 0.7.10⁸⁶ against the mm10/GRCm38 mouse reference and reads containing bases with quality lower than Q10 were removed. After alignment, reads were filtered out if it had more than one mismatch, more than 20As in a row (poly A tail), and aligned to more than 20 different positions in the transcriptome. Transcript quantifications were done using custom perl scripts and uniquely mapped reads with unique UMI's were processed for differential gene expression. Data analysis was performed using statistical software R version 3.3.3 and DESeq2 version 1.12.4⁸⁷. Differential genes were selected by ± 1.5-fold change. Heatmaps were generated using the GENE-E program and z-scores were calculated for each gene-row. GO terms were determined using Innate DB⁸⁸ and Pathway analysis was done using the Ingenuity Pathway Analysis tool (https://www.qiagenbioinformatics.com/). To define upstream regulators, log2(Fold Change) values were assigned to each gene in each comparison. Upstream regulators were then predicted by IPA software (Qiagen).

To analyze the cell death pathway, 1 µg of total RNA was pooled from the brain tissue of 4 newborn mice from each group (NLP or NLP_{ANT}). Gene expression was analyzed using the RT² Profiler PCR Array Mouse Cell Death Pathway Finder (cat. no. PAMM-212ZA- Qiagen) was used. Differential genes were selected by ± 2.0-fold change. Statistical analysis was performed using the RT2 profiler RT-PCR array data analysis software v3.5.

### Example 15: Nanoliposome construction.

Nanoliposomes were constructed using Sigma's Liposome kit (# L4395-5V) following the manufacturer's instructions. The nanoliposome preparations were serially extruded through a 200 nm and a 100 nm membrane and finally washed by ultracentrifugation. The nanoliposomes were resuspended in PBS and characterized before their use for their size (NLP 115.9±0.53, NLP_{ANT} 110.9±0.45), polydispersity index (NLP 0.066±0.003, NLP_{ANT} 0.082±0.003) and zeta potential (NLP 43±0.4, NLP_{ANT} 43.6±1.2).

### Example 16: In vivo ZIKV infection.

Eight to ten weeks old pregnant female SJL mice were intra-peritoneally infected with 10⁶ PFUs of ZIKV^{BR} (BeH815744) on E14 and followed daily. NLP_{ANT} were given daily from E13 until birth at 100 µg/animal also by i.p. route., receiving approximately 7 µg/animal of CH223191. HP163 was administered twice a day with 50 µg/animal by gavage. Crown-rump, biparietal, skull length, cranial height measurements were performed at E19 for HP163 and at the day of birth for NLPs. Neonates were measured using a pachymeter and weighted with a digital scale. All the experiments were performed with the approval of the Institute of Biomedical Sciences Ethics Committee - University of Sao Paulo.

### Example 17: Brain Histology.

Brains were removed from the skull and fixed in ice-cold 4% paraformaldehyde in 0.1 phosphate buffer pH 7.4 overnight, and cryoprotected in a solution of 30% sucrose in 0.1M phosphate buffer pH 7.4 at 4 °C. Samples were then frozen on dry ice, sectioned in the coronal plane at 20 µm thickness in a Leica semiautomatic cryostat (model 1850 UV, Leica Microsystems, Wetzlar, Germany) and mounted in super frost plus slides (VWR International, LLC Radnor, PA). Slides were dried for 48 hours, washed in distilled water for 2 minutes, incubated in Hematoxylin of Harris for 2 minutes, and then washed in running water to remove excess for 4 minutes. Slides were then incubated in Eosin for 2 minutes, dehydrated in increasing ethanol concentrations and diaphanization with xylol solution for 5 minutes and cover slipped with D.P.X. mounting medium (Sigma Aldrich, San Luis, MO, EUA). Images were acquired with an EVOS XL Core microscope (Thermo Fisher Scientific, MA, EUA) and analyzed by Image J Software.

### Example 18: Immunofluorescence.

Super frost plus mounted slides were blocked with blocking serum (3% donkey serum + 0,3% Triton X-100 in PBS) for 1 hour at room temperature. Sections were incubated with primary antibodies: Anti-pan flaviviruses Env (4G2) at 1:200 (anti-Rb GeneTex, Irvine, CA, USA); Anti-Nestin, 1:200 (anti-Gt, Santa Cruz Biotechnology, Dallas, TX, USA); and Anti-IBA1 (anti-Gt, Abcam, MA, USA) diluted in blocking serum, overnight at 4 °C. After incubation with the primary antibody, slides were incubated with secondary antibody (donkey anti-rabbit IgG, 488 and donkey anti-goat IgG, 594 Life technology, Carlsbad, CA, EUA; dilution 1:1000) diluted in PBS + 0,3% Triton X-100 for 2 hours at room temperature, protected from light. Slides were stained with DAPI for 20 minutes, 1:100.000 diluted in PBS + 0,3% Triton X-100 and coverslipped with Fluoromount G (Thermo Fisher, Waltham, MS, EUA) mounting medium. Images were acquired with a Nikon Eclipse 80i microscope (Nikon Instruments Inc., NY, USA) and analyzed by NIS- Elements Advanced Research 2.30 Image Software (Nikon Instruments Inc.).

### Example 19: Cells and Transient Transfection.

MOC1 ⁸⁹ were the generous gift of Dr. R. Uppaluri. SUM149 cells (Asterand Bioscience, Detroit, MI) were co-transfected with the pGudluc reporter plasmid (0.5 µg) (generously provided by Dr. M. Denison, UC, Davis), and CMV-green (0.1 µg) for normalization using TransIT-2020 transfection reagent (Mirus, Madison, WI). Transfection medium was replaced after 24 hours. The cells were left untreated or dosed with vehicle (DMSO or AHR inhibitors) and harvested after 24 hours in Glo Lysis Buffer (Promega, San Luis Obispo, CA). Luciferase activity was determined with the Bright-Glo Luciferase System according to the manufacturer's instructions (Promega). Luminescence and fluorescence were determined using a Synergy2 multifunction plate reader (Bio-Tek, Winooski, VT). pGudLuc luminescence was normalized to the CMV-green signal.

### Example 20: Protein isolation and Western blotting.

MOC1 and SUM149 cells were plated at 2 x 10 6 cells in T75 flasks and allowed to adhere overnight. Cells were pre-treated with 0.1% DMSO, 20 µM HP163 for 30 min and then treated with 0.1% DMSO, 100 µM nurenine, or 1 nM TCDD for 1 hour. Nuclear and cytoplasmic cell extracts were prepared using a Nuclear Extract Kit (Thermo Scientific, Rockford, IL) as per the manufacturer's instructions. Protein concentration was quantified using the Protein Assay Reagent (Bio-Rad, Hercules, CA). Protein (30 µg) was resolved on 10% SDS-polyacrylamide gels and transferred to nitrocellulose membranes (Bio-Rad). Membranes were probed with mouse anti-AHR (Pierce, cat #MA1-514, Rockford, IL), rabbit anti-Lamin-A/C (Cell Signaling, cat #2032, Danvers, MA) and mouse anti-α-tubulin (EMD Millipore, cat #CP06, Billerica, MA). Immuno-reactive bands were detected using HRP-conjugated secondary antibodies (goat anti-rabbit, Bio-Rad; goat anti-mouse Pierce) and ECL substrate.

### Example 21: Statistical analysis.

GraphPad Prism software version 6 was used for statistical analysis. Differences between groups was tested using student's t-test or one-way ANOVA followed by Tukey's post-hoc test as indicated in each experiment. A p value < 0.05 was considered significant. * P < 0.05; ** P < 0.01; *** P < 0.001.

SEQUENCE LISTING

<110> Hercules BV et al.

<120> TREATMENT OF CONGENITAL ZIKA VIRUS SYNDROME

<130> 79993EP9

<160> 10

<170> PatentIn version 3.5

<210> 1

<211> 21

<212>  DNA
<213>  Homo sapiens
<400>  1
cgtaagtcag cggtaggtct g                                                21

<210>  2
<211>  20
<212>  DNA
<213>  Homo sapiens
<400>  2
agaggccgac tgtgggtttt                                                  20

<210>  3
<211>  20
<212>  DNA
<213>  Homo sapiens
<400>  3
cagctgtggg ctctcctttc                                                  20

<210>  4
<211>  21
<212>  DNA
<213>  Homo sapiens
<400>  4
tacggtaaag cgggagaggt a                                                21

<210>  5
<211>  21
<212>  DNA
<213>  Homo sapiens
<400>  5
tgacatgctt ttccattggc g                                                21

<210>  6
<211>  20
<212>  DNA
<213>  Homo sapiens

<400>  6
agcaggatga tgccttgagt                                                   20

<210>  7
<211>  21
<212>  DNA
<213>  Homo sapiens
<400>  7
agatggctgc ccaccatatt t                                                 21

<210>  8
<211>  21
<212>  DNA
<213>  Homo sapiens
<400>  8
tgatagggcc tggctcttgt a                                                 21

<210>  9
<211>  21
<212>  DNA
<213>  Homo sapiens
<400>  9
cccaccacct acaaccctaa a                                                 21

<210>  10
<211>  20
<212>  DNA
<213>  Homo sapiens
<400>  10
cattctggaa aggagagccc                                                   20

### References:

1. Baud D, Gubler DJ, Schaub B, Lanteri MC, Musso D. An update on Zika virus infection. The Lancet 2017, 390(10107): 2099-2109.
2. Petersen LR, Jamieson DJ, Powers AM, Honein MA. Zika Virus. New England Journal of Medicine 2016, 374(16): 1552-1563.
3. MacNamara FN. Zika virus : A report on three cases of human infection during an epidemic of jaundice in Nigeria. Transactions of the Royal Society of Tropical Medicine and Hygiene 1954, 48(2): 139-145.
4. Musso D, Gubler DJ. Zika Virus. Clinical Microbiology Reviews 2016, 29(3): 487-524.
5. Faria NR, Azevedo R, Kraemer MUG, Souza R, Cunha MS, Hill SC, et al. Zika virus in the Americas: Early epidemiological and genetic findings. Science 2016, 352(6283): 345-349.
6. Faria NR, Quick J, Claro IM, Theze J, de Jesus JG, Giovanetti M, et al. Establishment and cryptic transmission of Zika virus in Brazil and the Americas. Nature 2017, 546(7658): 406-410.
7. Grubaugh ND, Ladner JT, Kraemer MUG, Dudas G, Tan AL, Gangavarapu K, et al. Genomic epidemiology reveals multiple introductions of Zika virus into the United States. Nature 2017, 546(7658): 401-405.
8. França GVA, Schuler-Faccini L, Oliveira WK, Henriques CMP, Carmo EH, Pedi VD, et al. Congenital Zika virus syndrome in Brazil: a case series of the first 1501 livebirths with complete investigation. The Lancet 2016, 388(10047): 891-897.
9. Liu Y, Liu J, Du S, Shan C, Nie K, Zhang R, et al. Evolutionary enhancement of Zika virus infectivity in Aedes aegypti mosquitoes. Nature 2017, 545(7655): 482-486.
10. Yuan L, Huang X-Y, Liu Z-Y, Zhang F, Zhu X-L, Yu J-Y, et al. A single mutation in the prM protein of Zika virus contributes to fetal microcephaly. Science 2017, 358(6365): 933-936.
11. Cao-Lormeau VM, Blake A, Mons S, Lastere S, Roche C, Vanhomwegen J, et al. Guillain-Barre Syndrome outbreak associated with Zika virus infection in French Polynesia: a case-control study. Lancet 2016, 387(10027): 1531-1539.
12. Boldescu V, Behnam MAM, Vasilakis N, Klein CD. Broad-spectrum agents for flaviviral infections: dengue, Zika and beyond. Nature Reviews Drug Discovery 2017, 16(8): 565-586.
13. Yan N, Chen ZJ. Intrinsic antiviral immunity. Nature Immunology 2012, 13(3): 214-222.
14. Perelygin AA, Scherbik SV, Zhulin IB, Stockman BM, Li Y, Brinton MA. Positional cloning of the murine flavivirus resistance gene. Proceedings of the National Academy of Sciences 2002, 99(14): 9322-9327.
15. Jiang D, Weidner JM, Qing M, Pan XB, Guo H, Xu C, et al. Identification of Five Interferon-Induced Cellular Proteins That Inhibit West Nile Virus and Dengue Virus Infections. Journal of Virology 2010, 84(16): 8332-8341.
16. Giovannoni F, Ladelfa MF, Monte M, Jans DA, Hemmerich P, Garcia C. Dengue Non-structural Protein 5 Polymerase Complexes With Promyelocytic Leukemia Protein (PML) Isoforms III and IV to Disrupt PML-Nuclear Bodies in Infected Cells. Frontiers in Cellular and Infection Microbiology 2019, 9(284).
17. Macnamara FN. Zika virus: a report on three cases of human infection during an epidemic of jaundice in Nigeria. Trans R Soc Trop Med Hyg 1954, 48(2): 139-145.
18. Liang Q, Luo Z, Zeng J, Chen W, Foo S-S, Lee S-A, et al. Zika Virus NS4A and NS4B Proteins Deregulate Akt-mTOR Signaling in Human Fetal Neural Stem Cells to Inhibit Neurogenesis and Induce Autophagy. Cell Stem Cell 2016, 19(5): 663-671.
19. Roth H, Magg V, Uch F, Mutz P, Klein P, Haneke K, et al. Flavivirus Infection Uncouples Translation Suppression from Cellular Stress Responses. MBio 2017, 8(1).
20. Tang H, Hammack C, Ogden Sarah C, Wen Z, Qian X, Li Y, et al. Zika Virus Infects Human Cortical Neural Progenitors and Attenuates Their Growth. Cell Stem Cell 2016, 18(5): 587-590.
21. Gutierrez-Vazquez C, Quintana FJ. Regulation of the Immune Response by the Aryl Hydrocarbon Receptor. Immunity 2018, 48(1): 19-33.
22. Bessede A, Gargaro M, Pallotta MT, Matino D, Servillo G, Brunacci C, et al. Aryl hydrocarbon receptor control of a disease tolerance defence pathway. Nature 2014, 511(7508): 184-190.
23. Opitz CA, Litzenburger UM, Sahm F, Ott M, Tritschler I, Trump S, et al. An endogenous tumour-promoting ligand of the human aryl hydrocarbon receptor. Nature 2011, 478(7368): 197-203.
24. Qian X, Nguyen HN, Song MM, Hadiono C, Ogden SC, Hammack C, et al. Brain-Region-Specific Organoids Using Mini-bioreactors for Modeling ZIKV Exposure. Cell 2016, 165(5): 1238-1254.
25. Chavali PL, Stojic L, Meredith LW, Joseph N, Nahorski MS, Sanford TJ, et al. Neurodevelopmental protein Musashi-1 interacts with the Zika genome and promotes viral replication. Science 2017, 357(6346): 83-88.
26. Cugola FR, Fernandes IR, Russo FB, Freitas BC, Dias JLM, Guimarães KP, et al. The Brazilian Zika virus strain causes birth defects in experimental models. Nature 2016, 534(7606): 267-271.
27. Martinot AJ, Abbink P, Afacan O, Prohl AK, Bronson R, Hecht JL, et al. Fetal Neuropathology in Zika Virus-Infected Pregnant Female Rhesus Monkeys. Cell 2018, 173(5): 1111-1122.e1110.
28. Retallack H, Di Lullo E, Arias C, Knopp KA, Laurie MT, Sandoval-Espinosa C, et al. Zika virus cell tropism in the developing human brain and inhibition by azithromycin. Proceedings of the National Academy of Sciences 2016, 113(50): 14408-14413.
29. Yockey LJ, Varela L, Rakib T, Khoury-Hanold W, Fink SL, Stutz B, et al. Vaginal Exposure to Zika Virus during Pregnancy Leads to Fetal Brain Infection. Cell 2016, 166(5): 1247-1256.e1244.
30. Gutiérrez-Vázquez C, Quintana FJ. Regulation of the Immune Response by the Aryl Hydrocarbon Receptor. Immunity 2018, 48(1): 19-33.
31. Kim SH, Henry EC, Kim DK, Kim YH, Shin KJ, Han MS, et al. Novel Compound 2-Methyl-2H-pyrazole-3-carboxylic Acid (2-methyl-4-o-tolylazo-phenyl)-amide (CH-223191) Prevents 2,3,7,8-TCDD-Induced Toxicity by Antagonizing the Aryl Hydrocarbon Receptor. Molecular Pharmacology 2006, 69(6): 1871-1878.
32. Rothhammer V, Borucki DM, Tjon EC, Takenaka MC, Chao C-C, Ardura-Fabregat A, et al. Microglial control of astrocytes in response to microbial metabolites. Nature 2018, 557(7707): 724-728.
33. Rothhammer V, Mascanfroni ID, Bunse L, Takenaka MC, Kenison JE, Mayo L, et al. Type I interferons and microbial metabolites of tryptophan modulate astrocyte activity and central nervous system inflammation via the aryl hydrocarbon receptor. Nat Med 2016, 22(6): 586-597.
34. Richardson RB, Ohlson MB, Eitson JL, Kumar A, McDougal MB, Boys IN, et al. A CRISPR screen identifies IFI6 as an ER-resident interferon effector that blocks flavivirus replication. Nature microbiology 2018, 3(11): 1214-1223.
35. Elong Ngono A, Shresta S. Immune Response to Dengue and Zika. Annual Review of Immunology 2018, 36(1): 279-308.
36. Lazear Helen M, Govero J, Smith Amber M, Platt Derek J, Fernandez E, Miner Jonathan J, et al. A Mouse Model of Zika Virus Pathogenesis. Cell Host & Microbe 2016, 19(5): 720-730.
37. Miner Jonathan J, Cao B, Govero J, Smith Amber M, Fernandez E, Cabrera Omar H, et al. Zika Virus Infection during Pregnancy in Mice Causes Placental Damage and Fetal Demise. Cell 2016, 165(5): 1081-1091.
38. Pfeffer LM, Kim J-G, Pfeffer SR, Carrigan DJ, Baker DP, Wei L, et al. Role of Nuclear Factor-κB in the Antiviral Action of Interferon and Interferon-regulated Gene Expression. Journal of Biological Chemistry 2004, 279(30): 31304-31311.
39. Rothhammer V, Quintana FJ. The aryl hydrocarbon receptor: an environmental sensor integrating immune responses in health and disease. Nature reviews Immunology 2019, 19(3): 184-197.
40. Yamada T, Horimoto H, Kameyama T, Hayakawa S, Yamato H, Dazai M, et al. Constitutive aryl hydrocarbon receptor signaling constrains type I interferon-mediated antiviral innate defense. Nature Immunology 2016, 17(6): 687-694.
41. Zhou Q, Lavorgna A, Bowman M, Hiscott J, Harhaj EW. Aryl Hydrocarbon Receptor Interacting Protein Targets IRF7 to Suppress Antiviral Signaling and the Induction of Type I Interferon. Journal of Biological Chemistry 2015, 290(23): 14729-14739.
42. Diaz MO, Ziemin S, Le Beau MM, Pitha P, Smith SD, Chilcote RR, et al. Homozygous deletion of the alpha- and beta 1-interferon genes in human leukemia and derived cell lines. Proceedings of the National Academy of Sciences 1988, 85(14): 5259-5263.
43. Dixit E, Boulant S, Zhang Y, Lee ASY, Odendall C, Shum B, et al. Peroxisomes Are Signaling Platforms for Antiviral Innate Immunity. Cell 2010, 141(4): 668-681.
44. Franz KM, Neidermyer WJ, Tan Y-J, Whelan SPJ, Kagan JC. STING-dependent translation inhibition restricts RNA virus replication. Proceedings of the National Academy of Sciences 2018, 115(9): E2058-E2067.
45. Moy Ryan H, Cole Brian S, Yasunaga A, Gold B, Shankarling G, Varble A, et al. Stem-Loop Recognition by DDX17 Facilitates miRNA Processing and Antiviral Defense. Cell 2014, 158(4): 764-777.
46. Giovannoni F, Damonte EB, Garcia CC. Cellular Promyelocytic Leukemia Protein Is an Important Dengue Virus Restriction Factor. PLOS ONE 2015, 10(5): e0125690.
47. Hubackova S, Krejcikova K, Bartek J, Hodny Z. Interleukin 6 Signaling Regulates Promyelocytic Leukemia Protein Gene Expression in Human Normal and Cancer Cells. Journal of Biological Chemistry 2012, 287(32): 26702-26714.
48. Stanford EA, Wang Z, Novikov O, Mulas F, Landesman-Bollag E, Monti S, et al. The role of the aryl hydrocarbon receptor in the development of cells with the molecular and functional characteristics of cancer stem-like cells. BMC Biol 2016, 14: 20.
49. Taguwa S, Maringer K, Li X, Bernal-Rubio D, Rauch JN, Gestwicki JE, et al. Defining Hsp70 Subnetworks in Dengue Virus Replication Reveals Key Vulnerability in Flavivirus Infection. Cell 2015, 163(5): 1108-1123.
50. Brass AL, Huang IC, Benita Y, John SP, Krishnan MN, Feeley EM, et al. The IFITM Proteins Mediate Cellular Resistance to Influenza A H1N1 Virus, West Nile Virus, and Dengue Virus. Cell 2009, 139(7): 1243-1254.
51. Manokaran G, Finol E, Wang C, Gunaratne J, Bahl J, Ong EZ, et al. Dengue subgenomic RNA binds TRIM25 to inhibit interferon expression for epidemiological fitness. Science 2015, 350(6257): 217-221.
52. Sessions OM, Barrows NJ, Souza-Neto JA, Robinson TJ, Hershey CL, Rodgers MA, et al. Discovery of insect and human dengue virus host factors. Nature 2009, 458(7241): 1047-1050.
53. Zhang R, Miner JJ, Gorman MJ, Rausch K, Ramage H, White JP, et al. A CRISPR screen defines a signal peptide processing pathway required by flaviviruses. Nature 2016, 535(7610): 164-168.
54. Marceau CD, Puschnik AS, Majzoub K, Ooi YS, Brewer SM, Fuchs G, et al. Genetic dissection of Flaviviridae host factors through genome-scale CRISPR screens. Nature 2016, 535(7610): 159-163.
55. Nganou-Makamdop K, Billingsley JM, Yaffe Z, O'Connor G, Tharp GK, Ransier A, et al. Type I IFN signaling blockade by a PASylated antagonist during chronic SIV infection suppresses specific inflammatory pathways but does not alter T cell activation or virus replication. PLoS pathogens 2018, 14(8): e1007246.
56. Sandler NG, Bosinger SE, Estes JD, Zhu RT, Tharp GK, Boritz E, et al. Type I interferon responses in rhesus macaques prevent SIV infection and slow disease progression. Nature 2014, 511(7511): 601-605.
57. Yeste A, Mascanfroni ID, Nadeau M, Burns EJ, Tukpah A-M, Santiago A, et al. IL-21 induces IL-22 production in CD4+ T cells. Nature Communications 2014, 5(1).
58. Mascanfroni ID, Takenaka MC, Yeste A, Patel B, Wu Y, Kenison JE, et al. Metabolic control of type 1 regulatory T cell differentiation by AHR and HIF1-α. Nature Medicine 2015, 21(6): 638-646.
59. Mascanfroni ID, Yeste A, Vieira SM, Burns EJ, Patel B, Sloma I, et al. IL-27 acts on DCs to suppress the T cell response and autoimmunity by inducing expression of the immunoregulatory molecule CD39. Nature Immunology 2013, 14(10): 1054-1063.
60. Gagliani N, Amezcua Vesely MC, Iseppon A, Brockmann L, Xu H, Palm NW, et al. Th17 cells transdifferentiate into regulatory T cells during resolution of inflammation. Nature 2015, 523(7559): 221-225.
61. Takenaka MC, Gabriely G, Rothhammer V, Mascanfroni ID, Wheeler MA, Chao CC, et al. Control of tumor-associated macrophages and T cells in glioblastoma via AHR and CD39. Nature neuroscience 2019, 22(5): 729-740.
62. Schiering C, Wincent E, Metidji A, Iseppon A, Li Y, Potocnik AJ, et al. Feedback control of AHR signalling regulates intestinal immunity. Nature 2017, 542(7640): 242-245.
63. Lawrence BP, Vorderstrasse BA. New insights into the aryl hydrocarbon receptor as a modulator of host responses to infection. Seminars in Immunopathology 2013, 35(6): 615-626.
64. Vaidyanathan B, Chaudhry A, Yewdell WT, Angeletti D, Yen W-F, Wheatley AK, et al. The aryl hydrocarbon receptor controls cell-fate decisions in B cells. The Journal of Experimental Medicine 2016, 214(1): 197-208.
65. Wheeler JLH, Martin KC, Lawrence BP. Novel Cellular Targets of AhR Underlie Alterations in Neutrophilic Inflammation and Inducible Nitric Oxide Synthase Expression during Influenza Virus Infection. The Journal of Immunology 2012, 190(2): 659-668.
66. Winans B, Nagari A, Chae M, Post CM, Ko C-I, Puga A, et al. Linking the Aryl Hydrocarbon Receptor with Altered DNA Methylation Patterns and Developmentally Induced Aberrant Antiviral CD8+T Cell Responses. The Journal of Immunology 2015, 194(9): 4446-4457.
67. Abbink P, Larocca RA, De La Barrera RA, Bricault CA, Moseley ET, Boyd M, et al. Protective efficacy of multiple vaccine platforms against Zika virus challenge in rhesus monkeys. Science 2016, 353(6304): 1129-1132.
68. Larocca RA, Abbink P, Peron JP, Zanotto PM, lampietro MJ, Badamchi-Zadeh A, et al. Vaccine protection against Zika virus from Brazil. Nature 2016, 536(7617): 474-478.
69. Sapparapu G, Fernandez E, Kose N, Bin C, Fox JM, Bombardi RG, et al. Neutralizing human antibodies prevent Zika virus replication and fetal disease in mice. Nature 2016, 540(7633): 443-447.
70. Safe S, Cheng Y, Jin UH. The Aryl Hydrocarbon Receptor (AhR) as a Drug Target for Cancer Chemotherapy. Curr Opin Toxicol 2017, 2: 24-29.
71. Boitano AE, Wang J, Romeo R, Bouchez LC, Parker AE, Sutton SE, et al. Aryl hydrocarbon receptor antagonists promote the expansion of human hematopoietic stem cells. Science 2010, 329(5997): 1345-1348.
72. Cheong JE, Sun L. Targeting the IDO1/TDO2-KYN-AhR Pathway for Cancer Immunotherapy - Challenges and Opportunities. Trends Pharmacol Sci 2018, 39(3): 307-325.
73. Brown JR, Conn KL, Wasson P, Charman M, Tong L, Grant K, et al. SUMO Ligase Protein Inhibitor of Activated STAT1 (PIAS1) Is a Constituent Promyelocytic Leukemia Nuclear Body Protein That Contributes to the Intrinsic Antiviral Immune Response to Herpes Simplex Virus 1. Journal of Virology 2016, 90(13): 5939-5952.
74. Geoffroy M-C, Chelbi-Alix MK. Role of Promyelocytic Leukemia Protein in Host Antiviral Defense. Journal of Interferon & Cytokine Research 2011, 31(1): 145-158.
75. Schilling E-M, Scherer M, Reuter N, Schweininger J, Muller YA, Stamminger T. The Human Cytomegalovirus IE1 Protein Antagonizes PML Nuclear Body-Mediated Intrinsic Immunity via the Inhibition of PML De Novo SUMOylation. Journal of Virology 2016, 91(4).
76. Ohtake F, Baba A, Takada I, Okada M, Iwasaki K, Miki H, et al. Dioxin receptor is a ligand-dependent E3 ubiquitin ligase. Nature 2007, 446(7135): 562-566.
77. Franchini AM, Lawrence BP. Environmental exposures are hidden modifiers of anti-viral immunity. Current Opinion in Toxicology 2018, 10: 54-59.
78. Thackray LB, Handley SA, Gorman MJ, Poddar S, Bagadia P, Briseño CG, et al. Oral Antibiotic Treatment of Mice Exacerbates the Disease Severity of Multiple Flavivirus Infections. Cell Reports 2018, 22(13): 3440-3453.e3446.
79. Manfredo Vieira S, Hiltensperger M, Kumar V, Zegarra-Ruiz D, Dehner C, Khan N, et al. Translocation of a gut pathobiont drives autoimmunity in mice and humans. Science 2018, 359(6380): 1156-1161.
80. Parks AJ, Pollastri MP, Hahn ME, Stanford EA, Novikov O, Franks DG, et al. In silico identification of an aryl hydrocarbon receptor antagonist with biological activity in vitro and in vivo. Mol Pharmacol 2014, 86(5): 593-608.
81. Bosch I, de Puig H, Hiley M, Carre-Camps M, Perdomo-Celis F, Narvaez CF, et al. Rapid antigen tests for dengue virus serotypes and Zika virus in patient serum. Sci Transl Med 2017, 9(409).
82. Chan JF, Yip CC, Tee KM, Zhu Z, Tsang JO, Chik KK, et al. Improved detection of Zika virus RNA in human and animal specimens by a novel, highly sensitive and specific real-time RT-PCR assay targeting the 5'-untranslated region of Zika virus. Trop Med Int Health 2017, 22(5): 594-603.
83. Lanciotti RS, Kosoy OL, Laven JJ, Velez JO, Lambert AJ, Johnson AJ, et al. Genetic and serologic properties of Zika virus associated with an epidemic, Yap State, Micronesia, 2007. Emerg Infect Dis 2008, 14(8): 1232-1239.
84. Ovcharenko I, Loots GG, Giardine BM, Hou M, Ma J, Hardison RC, et al. Mulan: multiple-sequence local alignment and visualization for studying function and evolution. Genome Res 2005, 15(1): 184-194.
85. Soumillon M, Cacchiarelli D, Semrau S, van Oudenaarden A, Mikkelsen TS. Characterization of directed differentiation by high-throughput single-cell RNA-Seq. Cold Spring Harbor Laboratory; 2014.
86. Li H, Durbin R. Fast and accurate short read alignment with Burrows-Wheeler transform. Bioinformatics 2009, 25(14): 1754-1760.
87. Love MI, Huber W, Anders S. Moderated estimation of fold change and dispersion for RNA-seq data with DESeq2. Genome Biology 2014, 15(12).
88. Breuer K, Foroushani AK, Laird MR, Chen C, Sribnaia A, Lo R, et al. InnateDB: systems biology of innate immunity and beyond-recent updates and continuing curation. Nucleic Acids Research 2012, 41(D1): D1228-D1233.
89. Judd NP, Winkler AE, Murillo-Sauca O, Brotman JJ, Law JH, Lewis JS, Jr., et al. ERK1/2 regulation of CD44 modulates oral cancer aggressiveness. Cancer research 2012, 72(1): 365-374.

SEQUENCE LISTING

<110>  Hercules Pharmaceuticals B.V. et al.

<120>  TREATMENT OF CONGENITAL ZIKA VIRUS SYNDROME

<130>  79993EP9

<160>  10

<170>  PatentIn version 3.5

<210>  1
<211>  21
<212>  DNA
<213>  Homo sapiens

<400>  1
cgtaagtcag cggtaggtct g                                                21


<210>  2
<211>  20
<212>  DNA
<213>  Homo sapiens

<400>  2
agaggccgac tgtgggtttt                                                  20


<210>  3
<211>  20
<212>  DNA
<213>  Homo sapiens

<400>  3
cagctgtggg ctctcctttc                                                  20


<210>  4
<211>  21
<212>  DNA
<213>  Homo sapiens

<400>  4
tacggtaaag cgggagaggt a                                                21


<210>  5
<211>  21
<212>  DNA
<213>  Homo sapiens

<400>  5
tgacatgctt ttccattggc g                                                21


<210>  6
<211>  20
<212>  DNA
<213>  Homo sapiens

<400>  6

agcaggatga tgccttgagt                                                    20

<210>  7
<211>  21
<212>  DNA
<213>  Homo sapiens

<400>  7
agatggctgc ccaccatatt t                                                  21

<210>  8
<211>  21
<212>  DNA
<213>  Homo sapiens

<400>  8
tgatagggcc tggctcttgt a                                                  21

<210>  9
<211>  21
<212>  DNA
<213>  Homo sapiens

<400>  9
cccaccacct acaaccctaa a                                                  21

<210>  10
<211>  20
<212>  DNA
<213>  Homo sapiens

<400>  10
cattctggaa aggagagccc                                                    20

## Claims

1. An antagonist of the aryl hydrocarbon receptor (AHR) or composition comprising such an antagonist or a pharmaceutically acceptable salt thereof for use in the treatment, amelioration or prevention of congenital ZIKA Virus (ZIKV) syndrome.

2. The antagonist or composition for use according to claim 1 wherein the congenital ZIKA virus syndrome is **characterized by** microcephaly and/or intra-uterine growth restriction (IUGR).

3. The antagonist or composition for use according to claim 1 or 2 wherein the antagonist has a structure according to formula 1: 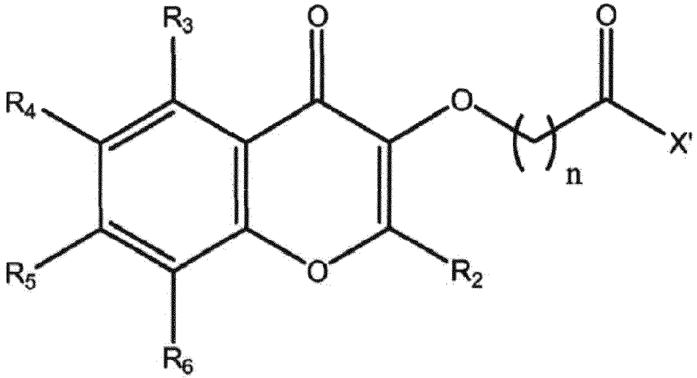 wherein:
X' is H, unsubstituted alkyl, aminosulfonyl, alkoxy, amino, acyl, aryl, or heteroaryl, each of which may be optionally substituted;
n is 0-6;
R2 is H, alkyl, acyl, heteroaryl, arylalkyl, cycloalkyl, heteroarylalkyl, heterocyclyl, or haloalkyl, each of which may be optionally substituted,
R3, R4, R5 and R6 are independently H, alkyl, acyl, halo, aryl, or heteroaryl, each of which may be optionally substituted, or
a stereoisomer.
